# EUROPEAN PATENT APPLICATION

(11) **EP 2 641 911 A2**
(43) Date of publication of application: **25.09.2013**
(21) Application number: 13156848.7
(22) Date of filing: 23.12.2009
(51) Int. Cl.: C07K 1/00, C07K 14/00, C07K 17/00, C12N 5/074, C12N 5/071

(54) **Compositions and methods for re-programming cells without genetic modification**

(30) Priority: 23.12.2008 US 203438 P; 19.03.2009 US 210586 P; 18.05.2009 US 216511 P; 17.07.2009 US 226659 P
(62) Divisional of application: 09835879.9
(71) Applicant: Vivoscript, Inc., Costa Mesa, CA 92626-1431 (US)
(72) Inventor: Zhu, Yong, Anaheim, CA 92807 (US); Wu, Shili, Edmond, OK 73012 (US); Bao, Jun, Rowland Heights, CA 91748 (US)
(74) Representative: Goodfellow, Hugh Robin

(57) **Abstract**

The present inventions are directed to compositions and methods regarding the reprogramming of biological samples (such as cells) without introducing exogenous genes to the samples. In particular, the present inventions are directed to transducible materials that are capable of transducing into the biological samples but are not genes or causing genetic modifications. The present inventions also are directed to methods of reprogramming the path of biological samples or treating diseases using the transducible compositions thereof.

## Description

### CROSS-REFERENCE TO RELATED APPLICATIONS

The present application claims priority to U.S. Provisional Application 61/203,438, filed 12/23/2008; U.S. Provisional Application 61/210,586, filed March 19, 2009; U.S. Provisional Application 61/216,511, filed May 18, 2009; and U.S. Provisional Application 61/226,659, filed July 17, 2009, all of which are incorporated herein by reference in their entirety.

### BACKGROUND OF THE INVENTION

Embryonic stem cells are capable of differentiating into many types of cells of human body. The majority of somatic cells are terminally differentiated and were believed to lack the capability of changing to other types of somatic cells. Recent advances in induced pluripotent stem cell (iPSC) and transdifferentiation fields have changed this paradigm. Somatic cells can be reprogrammed to induced pluripotent stem cell (iPSC), i.e. via ectopic expression of four transcription factors, *i.e.* Oct4 (e.g. SEQ ID NO: 1), Sox2 (e.g. SEQ ID NO: 2), Klf4 (SEQ ID NO: 3), and cMyc (e.g. SEQ ID NO: 4) via viral transduction (Okita et al., 2007; Takahashi and Yamanaka, 2006). A number of modified genetic approaches were further developed to produce iPSCs with potentially reduced risks, including using non-integrating adenoviruses to deliver reprogramming genes (Stadtfeld et al., 2008), transient transfection of reprogramming plasmids (Okita et al., 2008), a *piggyBac* transposition system and Cre-excisable viruses (Soldner et al., 2009; Woltjen et al., 2009). Furthermore, strategies of exploiting endogenous gene expression in certain cell types also allowed easier reprogramming and/or with less required exogenous genes (Aasen et al., 2008; Kim et al., 2008; Shi et al., 2008b). Moreover, small molecules have been identified that enhance reprogramming efficiency and replace certain reprogramming factors (Huangfu et al., 2008a; Huangfu et al., 2008b; Li et al., 2009; Shi et al., 2008a; Shi et al., 2008b). However, all of those methods to date still involve the use of genetic materials with drawbacks of introducing unknown, unwanted, or even harmful genome modifications by exogenous sequences in target cells and having inadequate control over expression levels of transgenes. To address these drawbacks, there are needs in the field to reprogram cells without relying upon or introducing exogenous genetic materials such as exogenous genes or DNA fragments or vector containing exogenous DNA or genes.

### BRIEF SUMMARY OF THE INVENTION

One aspect of the present disclosure relates to a transducible material comprising an effector domain. The effector domain is capable of exerting reprogramming changes of a biological sample once transduced into a biological sample. In certain embodiments, the effector domain is inherently capable of transducing into the biological sample.

In certain embodiments, the transducible material further comprises a transduction domain which is covalently or non-covalently associated with or linked to the effector domain. In certain embodiments, the transduction domain is covalently linked to the effector domain through a linker.

In certain embodiments, the transducible material is capable of selectively transducing into one or more specific biological samples or becoming transducible in a specific environment surrounding the biological sample.

Another aspect of the present disclosure relates to a composition comprising a biological sample and a transducible material, wherein the transducible material has transduced into the biological material.

Another aspect of the present disclosure relates to a method of reprogramming a biological sample by exposing the biological sample to a composition comprising a transducible material.

Another aspect of the present disclosure relates to a method of treating a disease or condition in a biological organism comprising administering a pharmaceutical composition comprising a transducible material into the biological organism.

Another aspect of the present disclosure relates to a method of developing cell-based therapies for various diseases or conditions comprising the step of reprogramming an iPSC, an embryonic stem cell, or a progenitor cell to a transplantable somatic cell or a transplantable progenitor cell using a transducible material.

Another aspect of the present disclosure relates to a method of developing disease models comprising the step of reprogramming an iPSC, an embryonic stem cell, or a progenitor cell to a transplantable somatic cell or a transplantable progenitor cell using a transducible material.

Another aspect of the present disclosure relates to a method of identifying an effector domain comprising covalently or non-covalently associating a test effector domain to a transduction domain to form a test transducible molecule, exposing the test molecule to a biological sample, and measuring a reprogramming level of the biological sample.

### BRIEF DESCRIPTION OF THE DRAWINGS

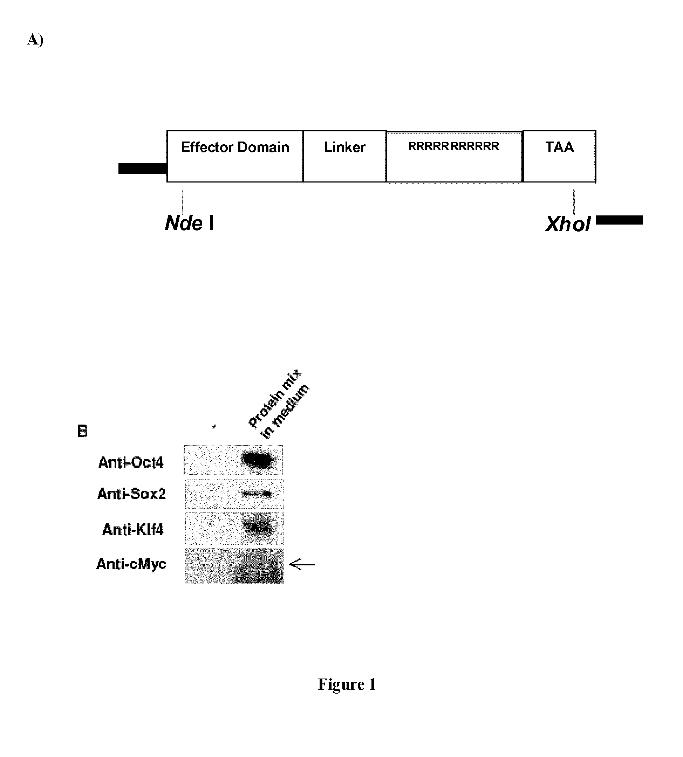

Figure 1: Characterization of transducible materials (I). (A) Schematic of protein expression vector for transducible materials Oct4-11R (SEQ ID NO: 12), Sox2-11R (SEQ ID NO: 13), Klf4-11R (SEQ ID NO: 14), and cMyc-11R (SEQ ID NO: 15), Linker: SEQ ID NO: 55; Effector Domain: Oct4 (SEQ ID NO: 1), Sox2 (SEQ ID NO: 2), Klf4 (SEQ ID NO: 3), or cMyc (SEQ ID NO: 4). (B) Stability of the four transducible materials (Oct4-11R , Sox2-11R, Klf4-11R, and cMyc-11R) under the cell culture condition examined by Western blot analysis.

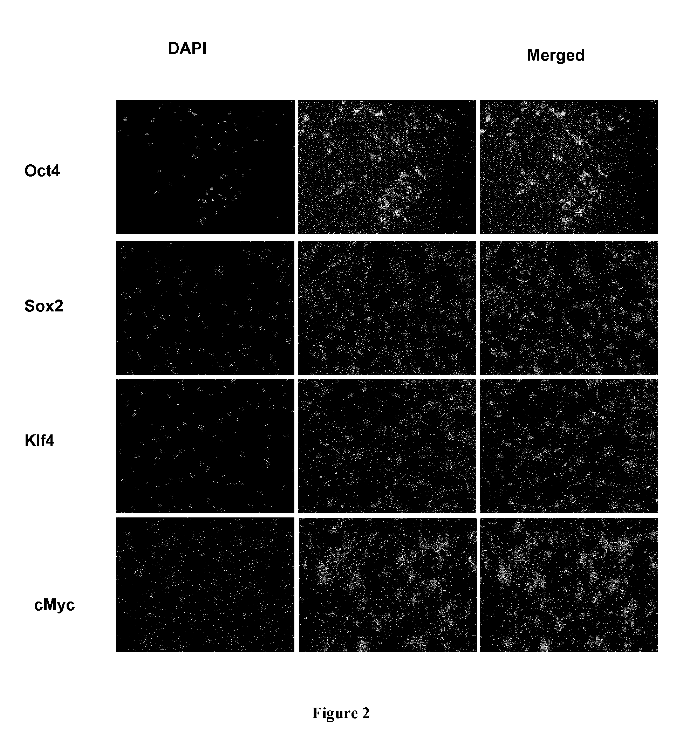

Figure 2: Characterization of transducible materials (II). Protein transduction of 11R-tagged transducible materials into OG2-MEF cells examined by immunocytochemistry. Oct4: MEF cells transuded with Oct4-11R (green), Sox2: MEF cells transuded with Sox2-11R (red), Klf4: MEF cells transuded with Klf4-11R (red) and cMyc: MEF cells transuded with cMyc-11R (green). DAPI: Cells stained with DAPI to visualize the nuclei (blue) and the images were merged.

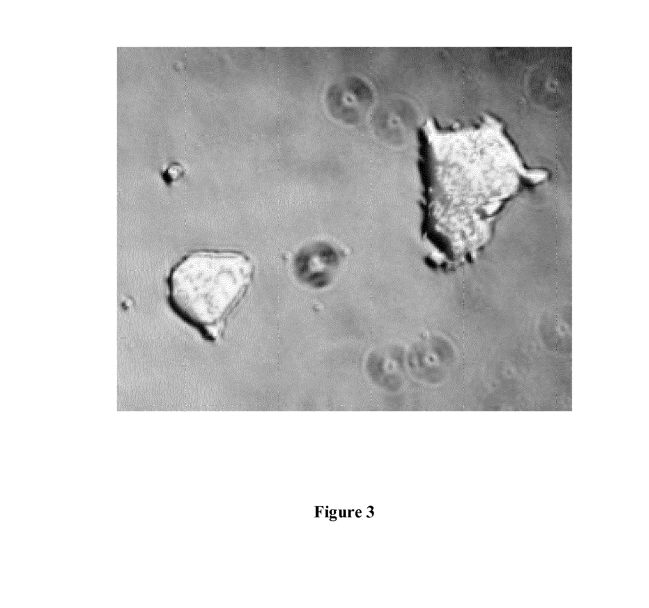

Figure 3: Characterization of transducible materials (III). Protein induced pluripotent stem (piPS) cells clonally expanded and self-renewed in chemical defined media and feeder free condition.

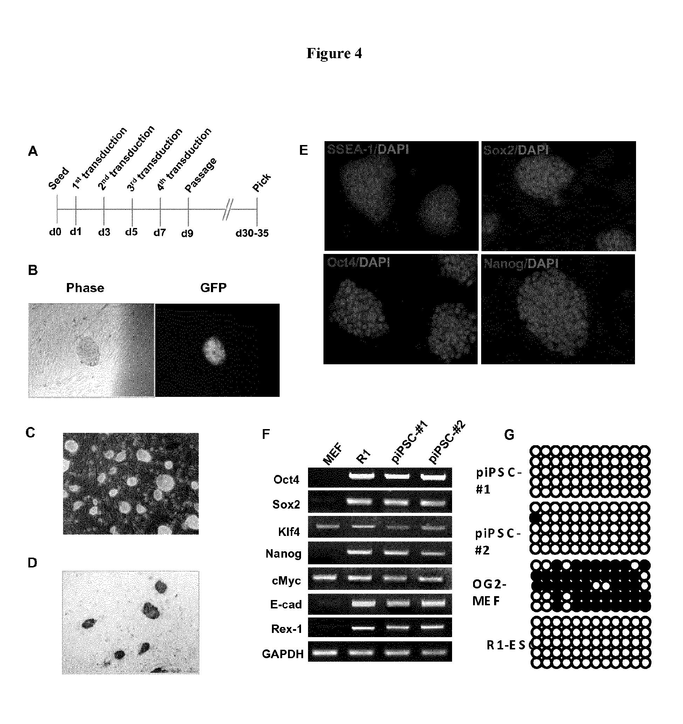

Figure 4: Generation of piPS cells by transducible materials Oct4-11R, Sox2-11R, Klf4-11R, and cMyc-11R. (A) Timeline of piPS cell generation. (B) Oct4-GFP⁺ piPS cell colonies initially observed around day 30-35. Phase: representative phase contrast image; and GFP: fluorescence image. (C) Oct4-GFP⁺ piPS cells sustained long term self-renewal under conventional mESC growth condition. (D) The long-term expanded piPS cells grew as compact and domed colonies that expressed strong ALP, a typical pluripotency marker. (E) piPS cells expressed other typical pluripotency markers, examined by immunofluorescence: SEA-1 (red), Sox2 (red), Oct4 (ed) and Nanog (red). DAPI: DAPI staining for visualization of the nuclei (blue), and the images were merged. (F) RT-PCR analysis of endogenous pluripotency gene expression in piPS cells. (G) Oct4 promoter methylation analysis by bisulfite genomic sequencing. Open and closed circles indicate unmethylated and methylated CpGs, respectively.

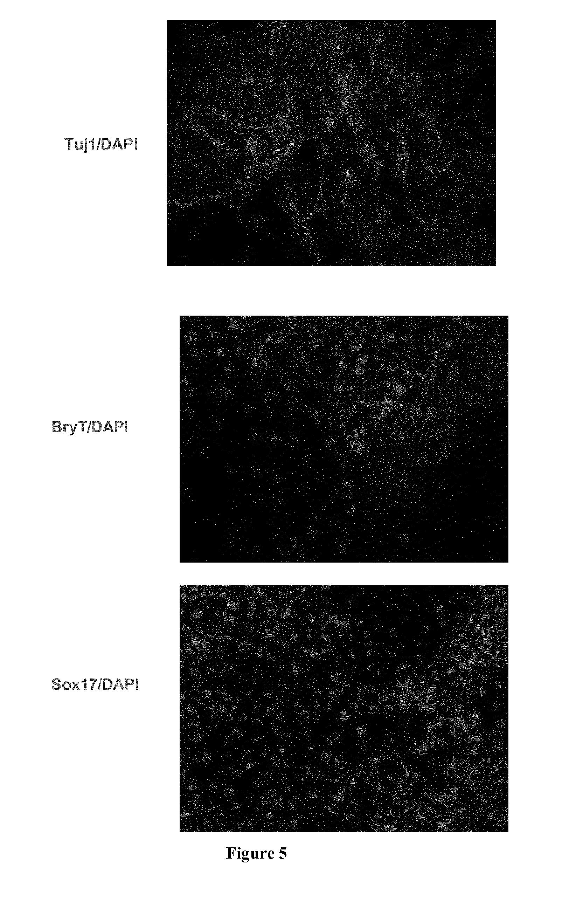

Figure 5: *In vitro* and *in vivo* pluripotency of piPS cells (I). piPS cells effectively differentiated *in vitro* into cells in the three germ layers: Tuj 1: characteristic TUJ1⁺ neuronal cells-ectoderm (red): Bryt: Brachyury⁺ mesoderm cells (red); and Soxl7: Soxl7⁺ definitive endoderm cells. Images were merged with DAPI (blue) staining.

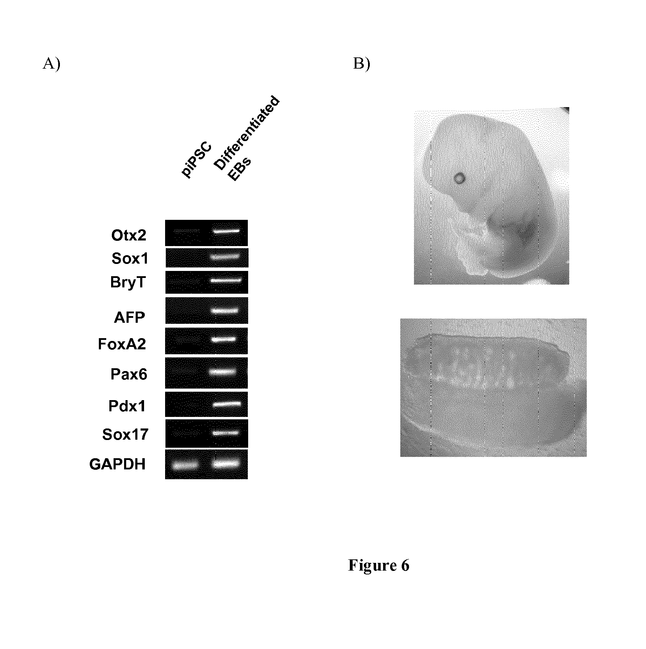

Figure 6: *In vitro* and *in vivo* pluripotency of piPS cells (II) (A) RT-PCR analysis of *in vitro* differentiation of piPS cells. (B) Chimeric embryos (13.5 dpc, 2 out of 7, left) obtained after transfer of the piPS cell aggregated embryos into a pseudo-pregnant mouse (top). Such piPS cells contributed to the germline cells (Oct4-GFP positive) in isolated genital ridge tissue from chimeric embryos (bottom).

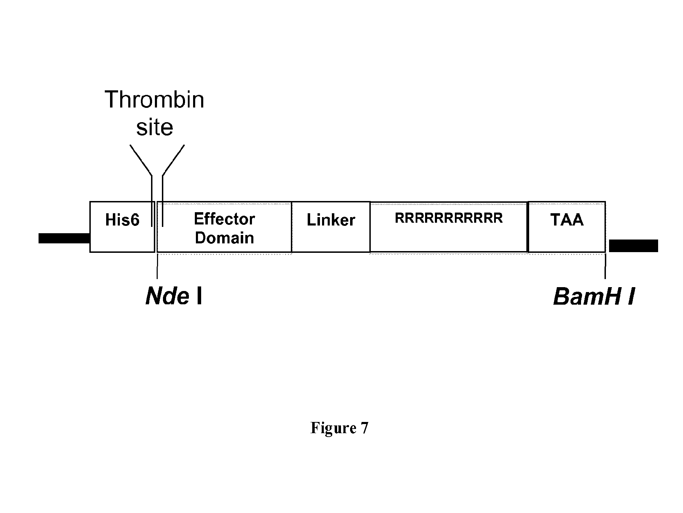

Figure 7: Schematic of protein expression vectors for transducible materials. His6: SEQ ID NO: 59; Effector Domain: Ngn3 (SEQ ID NO: 8), PDX1 (SEQ ID NO: 9); MafA (SEQ ID NO: 10), or Foxp3 (SEQ ID NO: 11); Linker: SEQ ID NO: 55.

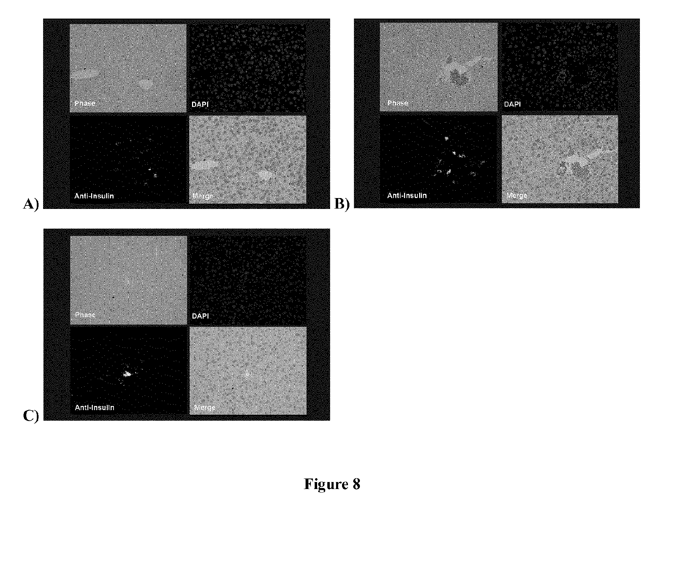

Figure 8: Reprogramming of liver and pancreatic exocrine cells to insulin-producing beta cells by transducible materials His6-Ngn3-11R (SEQ ID NO: 30), His6-PDX1-11R (SEQ ID NO: 31) and His6-MafA-11R (SEQ ID NO: 32) in mouse (I). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) Immunofluorescent analysis (IFA) of Mouse-1 liver; B) IFA of Mouse-2 liver; and C) IFA of Mouse-3 liver.

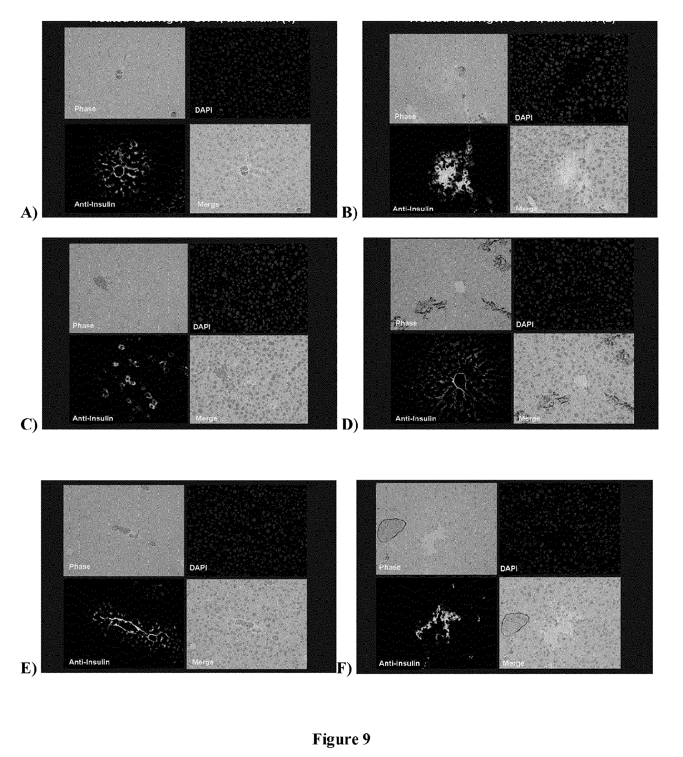

Figure 9: Reprogramming of liver and pancreatic exocrine cells to insulin-producing beta cells by transducible materials His6-Ngn3-11R, His6-PDX1-11R and His6-MafA-11R in mouse (II). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-4 liver (1); B) IFA of Mouse-4 liver (2); C) IFA of Mouse-5 liver (1); D) IFA of Mouse-5 liver (2); E) IFA of Mouse-6 liver (1); and F) IFA of Mouse-6 liver (2).

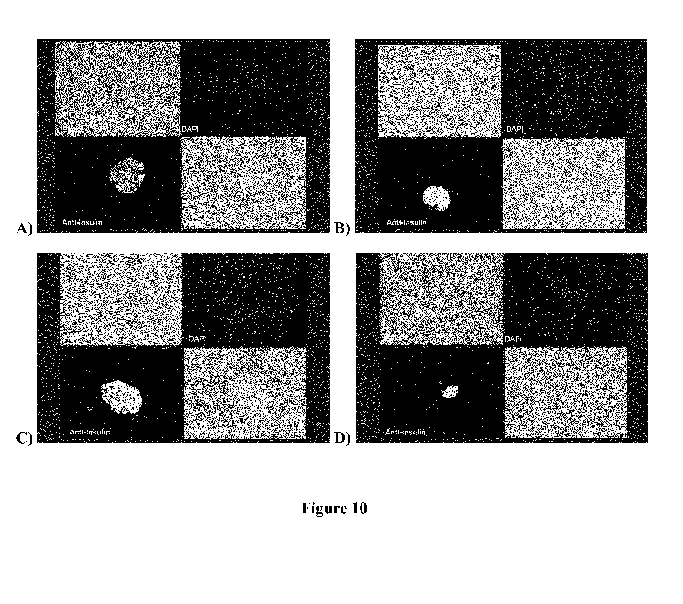

Figure 10: Reprogramming of liver and pancreatic exocrine cells to insulin-producing beta cells by transducible materials His6-Ngn3-11R, His6-PDX1-11R and His6-MafA-11R in mouse (III). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-1 pancrease; B) IFA of Mouse-2 pancrease (1); C) IFA of Mouse-2 pancrease (2); and D) IFA of Mouse-3 pancrease.

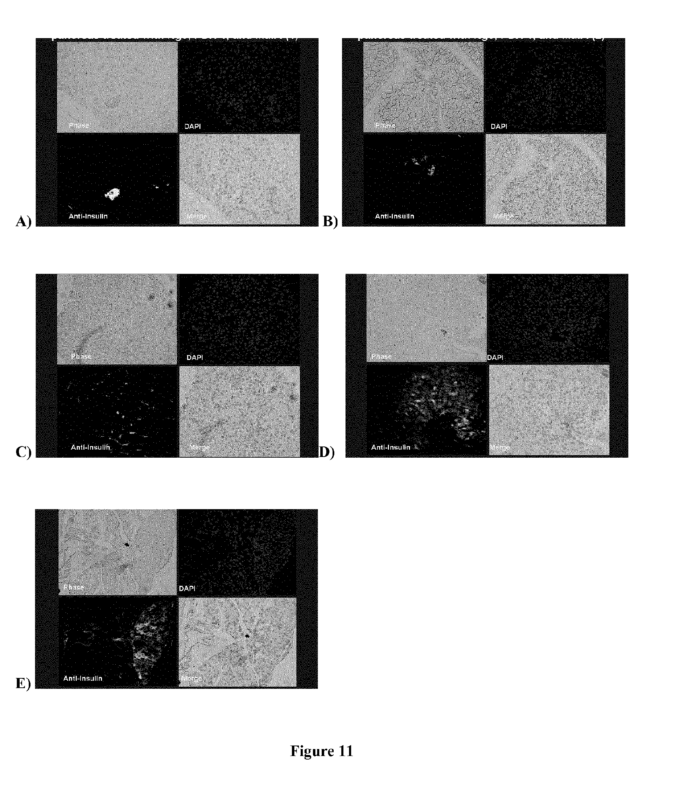

Figure 11: Reprogramming of liver and pancreatic exocrine cells to insulin-producing beta cells by transducible materials His6-Ngn3-11R, His6-PDX1-11R and His6-MafA-11R in mouse (IV). Mouse-1, Mouse-2 and Mouse-3 were treated with bovine serum albumin (BSA) protein (control group). Mouse-4, Mouse-5 and Mouse-6 were treated with His6-Ngn3-11R, His6-PDX1-11R and His6-MafA-11R (treatment group). A) IFA of Mouse-4 pancrease (1); B) IFA of Mouse-4 pancrease (2); C) IFA of Mouse-5 pancrease (1); D) IFA of Mouse-5 pancrease (2); and E) IFA of Mouse-6 pancrease.

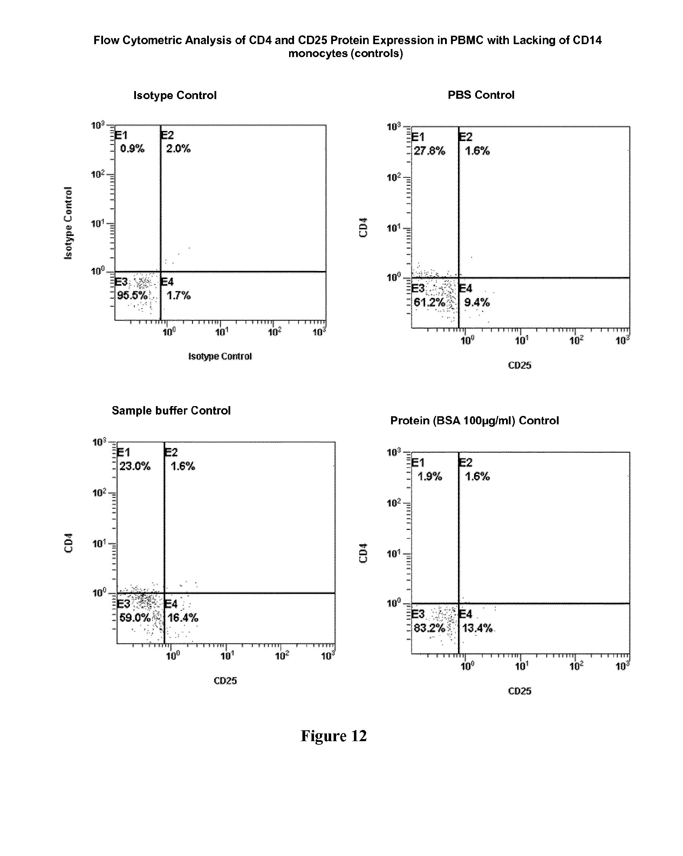

Figure 12: Reprogramming of T cells to Treg cells by transducible material His6-Foxp3-11R (SEQ ID NO: 33) (IA). Flow cytometric analysis of CD4 and CD25 protein expression in PBMC with lacking of CD14 monocytes: isotope control, PBS control, sample buffer control and protein (BSA 100µg/ml) control.

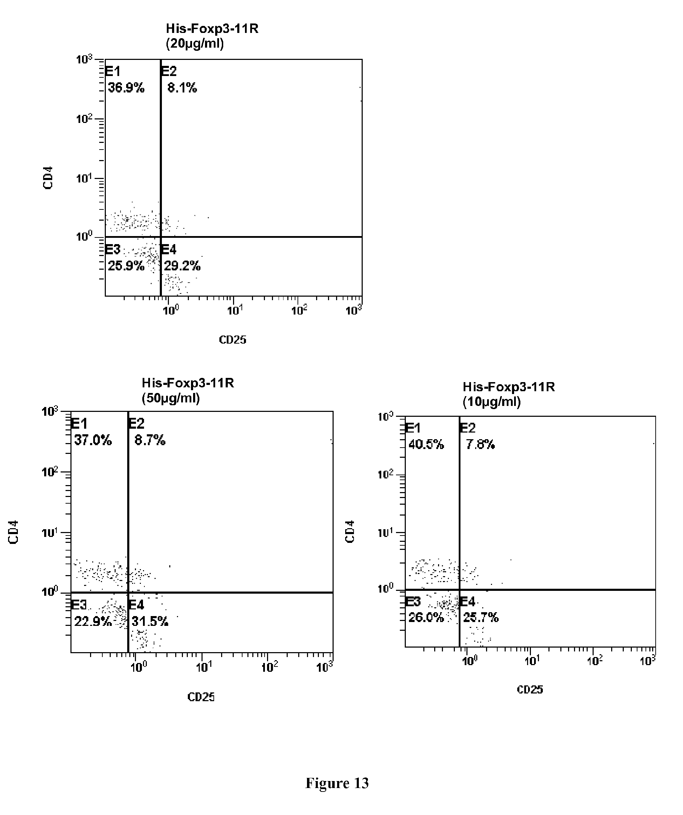

Figure 13: Reprogramming of T cells to Treg cells by transducible material His6-Foxp3-11R (IB). Flow cytometric analysis of CD4 and CD25 protein expression in PBMC with lacking of CD14 monocytes treated with His16-Foxp3-11R of 10µg/ml, 20 µg/ml, or 50 µg/ml.

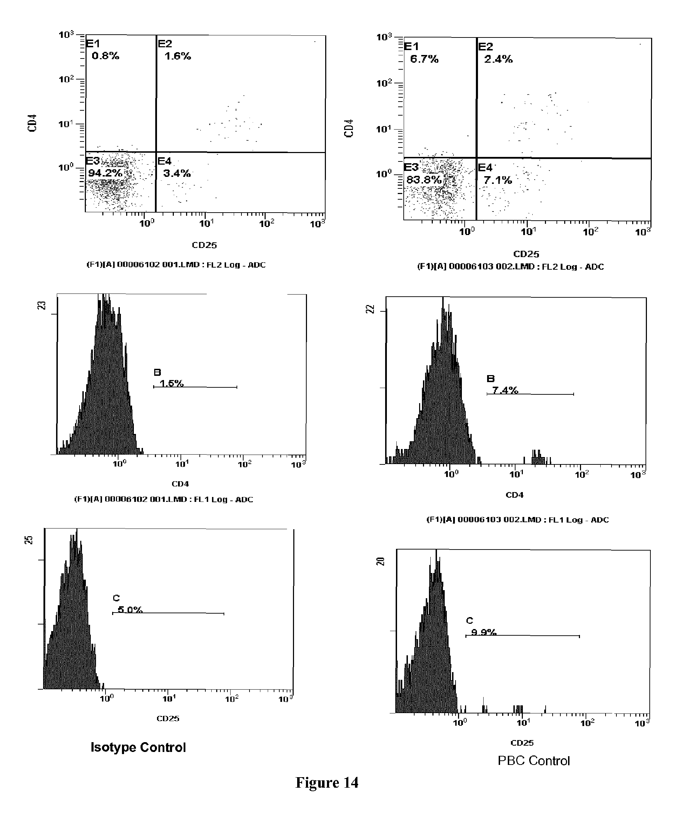

Figure 14: Reprogramming of T cells to Treg cells by transducible material His6-Foxp3-11R (IIA). Flow cytometric analysis of CD4 and CD25 protein expression in PBMC: isotope control, and PBS control.

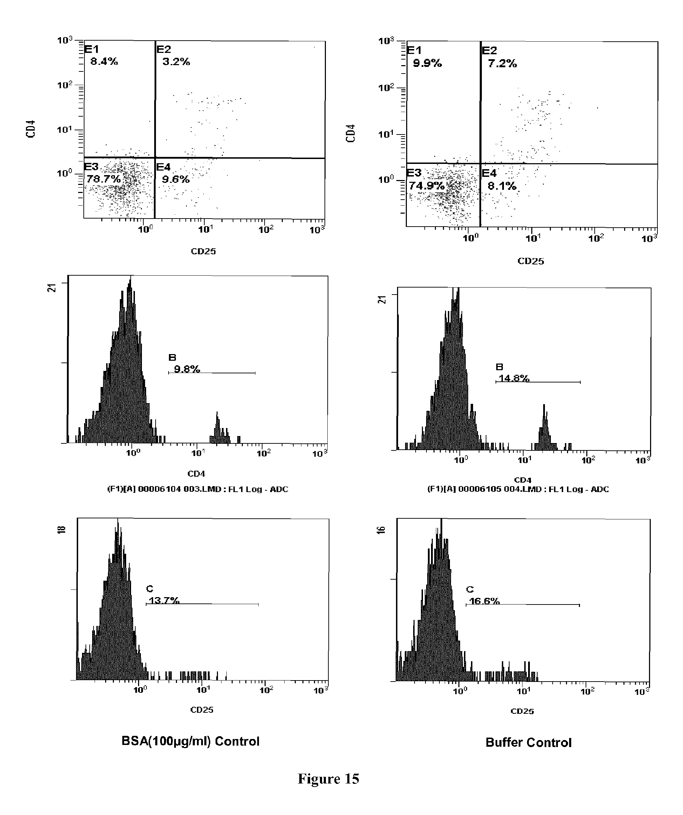

Figure 15: Reprogramming of T cells to Treg cells by transducible material His6-Foxp3-11R (IIB). Flow cytometric analysis of CD4 and CD25 protein expression in PBMC: sample buffer control and protein (BSA 100µg/ml) control.

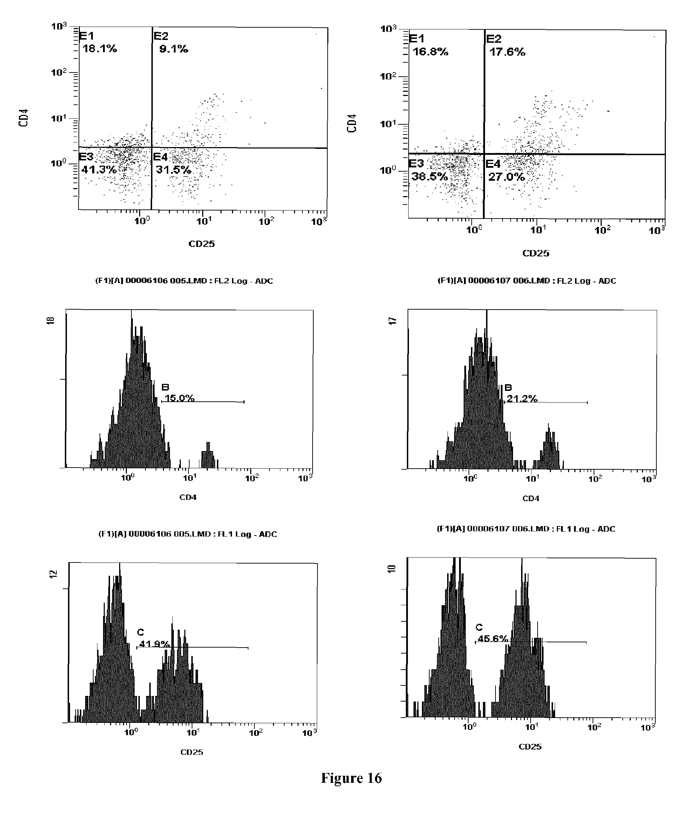

Figure 16: Reprogramming of T cells to Treg cells by transducible material His6-Foxp3-11R (IIC). Flow cytometric analysis of CD4 and CD25 protein expression in PBMC treated with His16-Foxp3-11R of 10µg/ml, or 50 µg/ml.

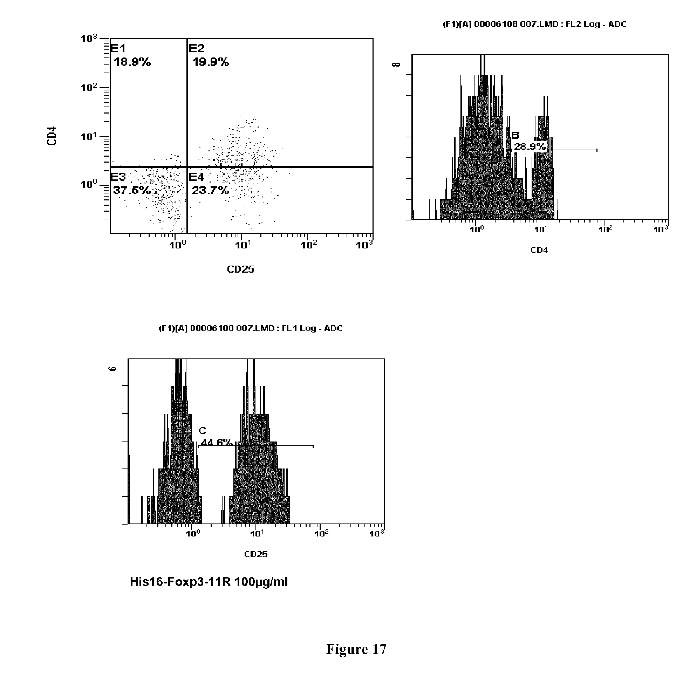

Figure 17: Reprogramming of T cells to Treg cells by transducible material His6-Foxp3-11R (IID). Flow cytometric analysis of CD4 and CD25 protein expression in PBMC treated with His16-Foxp3-11R of 100 µg/ml.

### DETAILED DESCRIPTION OF THE INVENTION

One aspect of the present disclosure relates to a transducible material comprising an effector domain.

In certain embodiments, a transducible material used herein refers to a material or a molecule which is not DNA or derived from DNA but is capable of crossing or transducing or being crossed through a membrane of a biological sample (e.g., a cell membrane) so that the transducible material can enter or be brought into the inside of the biological sample from the outside of the biological sample and exerts reprogramming efforts. For example, the transducible material may interact with cell-surface receptors which facilitate the entry of the material into cells through receptor mediated endocytosis.

In certain embodiments, a transducible material is a selective transducible material which is more likely to transduce into a specific type of biological samples (e.g. cancer or tumor cells) or becomes transducible in a specific microenvironment in or around a biological sample (e.g. microenvironment around cancer or tumor) than other biological samples. For example, the selective transducible material comprises a transduction domain (e.g. a cell-targeting peptide or an activatable cell penetrating peptide) that preferably delivers the selective transducible material into a specific type of biological sample or become transducible in a microenvironment around a biological sample.

Without being bounded to any theories, it is contemplated that the transducible materials may cross a cell membrane and enter into cytoplasm to reprogram cytoplasm activities such as translation, post-translation modification, signaling pathway, apoptosis pathway. It is further contemplated that the transducible material may cross the nucleus membrane and reprogram or modulate DNA or chromosomal replication, gene transcription, and RNA splicing.

An effector domain is a motif or a molecule which, once inside a biological sample, is capable of exerting reprogramming changes of the biological sample. The effector domain may interact with molecules (e.g., proteins, DNA, RNA, sugars, and lipids) in the biological sample (e.g., in cytoplasm or nuclei) and lead to changes such as proliferation, differentiation, dedifferentiation, transdifferentiation, retrodifferentiation, transdertermination, apoptosis, and morphogenesis. The effector domain can be 1) a polypeptide, or a fragment or a mimic thereof; 2) a polynucleotide which cannot be gene expressed once transduced or incorporated into the genome of the biological sample or cause genetic modification but nevertheless interacts with molecules in the biological sample (e.g., a ribozyme, an antisense molecule, a siRNA or miRNA, an oligonucleotide, and the like); and 3) a small molecule or other chemical compound (e.g. chemotherapy drugs).

In certain embodiments, an effector domain is inherently transducible, e.g. PDX1 (e.g. SEQ ID NO: 9) and NeuroD (e.g. SEQ ID NO: 7).

One example of the effector domain is a polypeptide such as a transcription factor, a chromosome remodeling protein, an antibody, or a fragment or mimic thereof. Another example of the effector domain is a small molecule which is not a polymer and binds with a biolpolymer such as protein, nucleic acid, or polysaccharide and alters the activity or function of the biopolymer. Examples of small molecules include, without limitation, acetylation inhibitors, transcription activators, signal pathway activators, signal pathway inhibitors, and methylation inhibitors.

In another embodiment, an effector domain can be at least one polypeptide that reprograms a somatic cell into a stem cell or change a cell state from one to another. For example, the effector domain can be 1) a polypeptide selected from the group consisting of Klf4 (e.g. SEQ ID NO: 3), Sox2 (e.g. SEQ ID NO: 2), Lin28 (e.g. SEQ ID NO: 5), Oct4 (e.g. SEQ ID NO: 1), cMyc (e.g. SEQ ID NO: 4), Nanog (e.g. SEQ ID NO: 6), and any combination thereof; 2) a polypeptide selected from the group consisting of Klf4, Sox2, Oct4, cMyc, and any combination thereof; 3) a polypeptide selected from the group consisting of Sox2, Oct4, Lin28, Nanog, and any combination thereof; 4) a polypeptide selected from the group consisting of Ngn3 (e.g. SEQ ID NO: 8), PDX1 (e.g. SEQ ID NO: 9), MafA (e.g. SEQ ID NO: 10), NeuroD (e.g. SEQ ID NO: 7), and any combination thereof; 5) a polypeptide comprising Foxp3 (e.g. SEQ ID NO: 11); 6) a polypeptide selected from the group consisting of Oct4, Sox2, Klf4, Lin28, Nanog, cMyc, Ngn3, PDX1, MafA, NeuroD, Foxp3, and any combination thereof; 7) a combination of polypeptides Oct4, Sox2, Klf4 and cMyc; and 8) a combination of polypeptides Ngn3, PDX1 and MafA.

In certain embodiments, a transducible material further comprises a transduction domain. A transduction domain is a motif that is capable of facilitating the entry of the transducible material into a biological sample (e.g., a cell). The transducible domain is associated with the effector domain covalently, noncovalently or via a linker. In certain embodiments, the transduction domain is covalently linked to the effector domain through a linker. In certain embodiments, the linker is a glycine-rich linker that comprises one or more glycine residues (e.g. esggggspg (SEQ ID NO: 55)).

Examples of a transduction domain include, without limitation, polymers such as cationic lipid polymers and nanoparticles, protein transduction domains (PTD), cell penetrating peptides (CPP1), cell permeating peptides (CPP2), activatable cell penetrating peptides or conjugates (ACPP), and cell-targeting peptides (CTP).

CPP1, CPP2, and PTD are peptides known to facilitate delivery of a molecular cargo associated thereof into cells. The association between a CPP1, CPP2 or PTD and the molecular cargo can be through covalent bond or non-covalent interactions. The molecular cargo can be small chemical molecules, peptides, protein, fragment of DNA, RNA such as siRNA and miRNA, or nanosize particles. For example, CPP1 and PTD include 5 to 20 amino acid peptide motifs that are capable of penetrating cells independent of surface transporters and of cell cycle phase. CPP1 and PTD can also be capable of penetrating through blood-brain barriers. CPP1 and PTD can deliver proteins and peptides *in vitro* and *in vivo* with uniform distribution throughout the organism after parenteral administration. Cationic PTDs can act as nuclear localization signals and carry an associated molecular cargo to cell nuclei. Examples of protein transduction domains include, without limitation, TAT (e.g. YGRKKRRQRRR, SEQ ID NO: 34), poly-arginine (e.g. poly-arginine having 7-11 arginine residues such as RRRRRRR, RRRRRRRR, RRRRRRRRR, RRRRRRRRRR (SEQ ID NO: 35)and RRRRRRRRRRR (SEQ ID NO: 36)), Penetratin (Antennapedia, e.g. RQIKIWFQNRRMKWKK (SEQ ID NO: 38)), VP22 (e.g. DAATATRGRSAASRPTQRPRAPARSASRPRRPVQ (SEQ ID NO: 39)), Transportan (e.g. GWTLNSAGYLLGKINLKALAALAKKIL (SEQ ID NO: 40)), MAP (e.g. KLALKLALKALKAALKLA (SEQ ID NO: 41)), MTS (e.g. AAVALLPAVLLALLP (SEQ ID NO: 42)), PEP-1 (e.g. KETWWETWWTEWSQPKKKRKV (SEQ ID NO: 43)), Arg/Trp analogue (e.g. RRWRRWWRRWWRRW (SEQ ID NO: 44)), polyguanidine peptoids (e.g. polyguanidine peptoids with a 6-methylene spacer between backbone and guanidine group such as N-arg 5, 7 or 9 peptoids), HIV-1 Rev (e.g. SEQ ID NO: 60), Flock house virus coat peptide (e.g. SEQ ID NO: 61), and DNA-binding peptides such as c-Fos (e.g. SEQ ID NO: 62), c-Jun (e.g. SEQ ID NO: 63)and yeast GCN4 (e.g. SEQ ID NO: 64).

Cell-targeting peptides are proteins or peptides that bind to cell-surface receptors and enter cells through endocytosis. In certain embodiments, a cell-target peptide targets specific tissues or cell types, for example, GnRH peptides (e.g. SEQ ID NO: 58) target biological samples that express GnRH receptors (e.g. solid tumors and hormone-responsive cancer cell lines). More examples of cell-targeting peptides and the specific biological samples targeted are listed in Table 1.

**Table 1. Examples of cell-targeting peptides and the specific biological samples targeted.**

| **Sequence ID No.** | **Peptide sequence** | **Length** | **Targeted tissue or cells and the cellular targets** |
|---|---|---|---|
| SEQ ID NO: 45 | TSPLNIHNGQKL | 12 | Human head and neck solid tumors |
| SEQ ID NO: 46 | CGKRK | 5 | Tumor neovasculature, Heparan sulfate |
| SEQ ID NO: 47 | CGNKRTRGC | 7 | breast carcinoma |
| SEQ ID NO: 48 | SMSIARL | 7 | Prostate vasculature |
| SEQ ID NO: 49 | FQHPSFI | 7 | hepatocellular carcinoma cell line |
| | RGD | 3 | Integrin receptor', αVβ3 |
| | NGR | 3 | Tumor neovasculature, Amino-peptidase N |
| SEQ ID NO: 50 | VHSPNKK | 7 | Endothelial VCAM-1 expressing cells; VCAM-1 |
| SEQ ID NO: 51 | RRPYIL | 6 | Adenocarcinoma cells; Neurotensin receptor |
| SEQ ID NO: 52 | EDYELMDLLAYL | 12 | Various carcinoma |
| SEQ ID NO: 53 | LTVSPWY | 7 | breast carcinoma; erbB2 |
| SEQ ID NO: 54 | ATWLPPR | 7 | Tumor neovasculature; VEGF receptor |

An activatable cell penetrating peptide or conjugate (ACPP) comprises a cationic CPP1, CPP2 or PTD and a neutralizing anionic counterpart. In certain embodiments, the cationic CPP1, CPP2 or PTD and the anionic counterpart are associated via noncovalent interactions (e.g. charge-charge interaction) and/or covalent cleavable linker (e.g. matrix metaloprotease (MMP) cleavable sequence). Transduction of an ACPP into cells are inhibited until the noncovalent interactions are disrupted and/or the cleavable linker is cleaved. For example, without being bound to any theory, the anionic counterparts comprise one or more pH sensitive groups such as sulfonamide groups, which are protonated at pH 7.4 (the pH in the blood stream) and become neutral at slightly acidic pH (e.g. pH 6.8). Therefore, charge-charge interactions between cationic CPP 1, CPP2 or PTD and the anionic counterpart can be interrupted in slightly acidic microenvironment (e.g. in or around tumor or cancer). MMP concentration in blood stream is lower than that in a microenvironment around tumor or cancer. Therefore, MMP cleavable sequence, which is not cleaved in the bloodstream, is cleaved in environment around tumor or cancer. The cationic CPP 1, CPP2 or PTD is no longer neutralized by the anionic counterpart, and therefore is exposed to facilitate the translocation into cells (e.g. tumor or cancer cells). In certain embodiments, the CPP1, CPP2 or PTD is TAT. In certain embodiments, the anionic counterparts comprise pH-sensitive polymer (e.g. di-block copolymer) comprising pH-sensitive groups (e.g. sulfonamide groups).

For another example, an activatable cell penetrating conjugate comprises a conventional hydrophobic core made of a polymer into which an effective domain is incorporated, a peripheral hydrophilic layer composed of poly-ethylene glycol and one or more cationic CPP1s, CPP2s or PTDs, and one or more anionic counterpart that neutralize the cationic CPP12, CPP2s or PTDs through charge-charge interactions. Such charge-charge interactions are expected to shield the cationic charges during delivery until the transduction material reaches a slightly acidic microenvironment (e.g. tumor or cancer), which triggers protonation of the anionic counterparts and disrupts the charge-charge association. Subsequently the cationic CPP1s, CPP2s, or PTDs, previously quenched by the anionic counterpart, are now capable of facilitating delivery of the effector domain into the surrounding cells (e.g. tumor or cancer cells).

In certain embodiments, a selective transducible material comprises a transduction domain selected from the group consisting of cell-targeting peptides and activatable cell penetrating peptides and activatable cell penetrating conjugates.

A transduction domain is associated to an effector domain via covalent bond, non-covalent interactions or through a linker. Thus a transducible material can be made by obtaining the transduction domain and the effector domain separately and associate together through a covalent bond or non-covalent interactions (e.g., repulsive interactions, dipole interactions, hydrogen bonding interactions, dispersive interactions, charge-charge interactions, solvent, counter ion and entropic effects, and water and hydrophobic effects). In certain embodiments, the transduction material is prepared by mixing the effector domain and transducible domain. Alternatively, a transducible material can be produced by isolating the material from natural resources or recombinantly. In the case when both domains are peptides or polypeptides, the effector domain can be linked to the N-terminus or C-terminus of the transduction domain and the transducible polypeptide can be made synthetically through chemical synthesis or recombinantly through recombinant technology.

In certain embodiments, a transducible material comprises an effector domain that is inherently transducible, and a transduction domain associated with the effector domain via covalent or non-covalent interactions.

In certain embodiments, a transducible material further comprises one or more motifs that do not interrupt the function of the effector domain or the transduction domain. In certain embodiments, these motifs are linked covalently, non-covalently or through a linker to the effector domain and/or the transduction domain. In certain embodiments, these motifs facilitate the preparation and/or purification of the transducible material. One example of such motif is a polyhistidine-tag to facilitate protein purification in preparation of the transducible material. In certain embodiments, the polyhistidine-tag comprises at least six histidine residues (e.g. MGSSHHHHHHSSGLVPRGSH ("His6," SEQ ID NO: 59)).

In certain embodiments, a transducible material includes, for example, Oct4-11R (SEQ ID NO: 12), Sox2-11R (SEQ ID NO: 13), Klf4-11R (SEQ ID NO: 14), Lin28-11R (SEQ ID NO: 16), Nanog-11R (SEQ ID NO: 17), cMyc-11R (SEQ ID NO: 15), Ngn3-11R (SEQ ID NO: 19), PDX1-11R (SEQ ID NO: 20), MafA-11R (SEQ ID NO: 21), NeuroD-11R (SEQ ID NO: 18), and Foxp3-11R (SEQ ID NO: 22), wherein 11R (SEQ ID NO: 37) stands for a polyarginine sequence of 11 arginine residues linking to a linker through which the polyarginie sequence is covalently linked to the effector domain. In certain embodiments, a transducible material includes, for example, His6-Oct4-11R (SEQ ID NO: 23), His6-Sox2-11R (SEQ ID NO: 24), His6-Klf4-11R (SEQ ID NO: 25), His6-Lin28-11R (SEQ ID NO: 27), His6-Nanog-11R (SEQ ID NO: 28), His6-cMyc-11R (SEQ ID NO: 26), His6-Ngn3-11R (SEQ ID NO: 30), His6-PDX1-11R (SEQ ID NO: 31), His6-MafA-11R (SEQ ID NO: 32), His6-NeuroD-11R (SEQ ID NO: 29), and His6-Foxp3-11R (SEQ ID NO: 33).

In certain embodiments, a transducible material can be combined with one or more adjuvants such as growth factors to stimulate cellular growth. For example, islet growth factor (e.g. betacellulin) is used as an adjuvant in reprogramming liver and/or pancreatic exocrine cells to insulin producing cells (e.g. β cells).

Another aspect of the present disclosure relates to a composition comprising a biological sample and at least one transducible material, wherein the transducible material has transduced into the biological sample. For example, the composition includes a transducible material comprising Foxp3 (e.g. Foxp3-11R and His6-Foxp3-11R) and a T cell wherein the transducible material has transduced into the T cell; a composition includes a piPS cell and one or more transducible materials comprising a polypeptide selected from the group consisting of Oct4, Klf4, Sox2 and cMyc, and any combination thereof (e.g. Oct4-11R, Klf4-11R, Sox2-11R, cMyc-11R, His6-Oct4-11R, His6-Klf4-11R, His6-Sox2-11R and His6-cMyc-11R); and a composition including a liver or pancreatic exocrine cell and one or more transducible materials comprising a polypeptide selected from the group consisting of Ngn3, PDX1, MafA, NeuroD, and any combination thereof (e.g. Ngn3-11R, PDX1-11R, MafA-11R, His6-Ngn3-11R, His6-PDX1-11R and His6-MafA-11R) wherein the transducible materials have transduced into the liver or pancreatic exocrine cell.

Another aspect of the present disclosure relates to a method of reprogramming a biological sample by exposing the biological sample to a composition comprising a transducible material. In certain embodiments, the method preferably reprograms a specific type of biological sample (e.g. cancer or tumor cells) or biological samples in or around a specific microenvironment within a biological organism (e.g. microenvironment around cancer or tumor) than other biological samples by exposing biological samples to a composition comprising a selective transducible material.

In one embodiment, a biological sample includes a cell, a cluster of cells, a tissue, an organ, a biological body from a biological organism. The biological sample can be normal, healthy sample or abnormal, diseased sample (e.g., cancer or tumor).

A biological organism includes, for example, a microorganism (e.g., bacteria), a fungus, a plant and an animal (e.g., a human).

An organ from an animal biological organism (e.g., human) includes, for example, a circulatory organ (e.g., heart, blood and blood vessels), a digestive organ (e.g., salivary glands, esophagus, stomach, liver, gallbladder, pancreas, intestines, rectum and anus), an endocrine organ (e.g., endocrine glands such as the hypothalamus, pituitary or pituitary gland, pineal body or pineal gland, thyroid, parathyroids and adrenals, i.e., adrenal glands), an integumentary organ (e.g., skin, hair and nails), a lymphatic organ (e.g., lymph nodes and vessels, tonsils, adenoids, thymus and spleen), a muscular organ (e.g., muscles), a nervous organ (e.g., brain, spinal cord, peripheral nerves and nerves), a reproductive organ (e.g., ovaries, fallopian tubes, uterus, vagina, mammary glands, testes, vas deferens, seminal vesicles, prostate and penis), a respiratory organ (e.g., the pharynx, larynx, trachea, bronchi, lungs and diaphragm), a skeletal organ (e.g., bones, cartilage, ligaments and tendons), a urinary system (e.g., kidneys, ureters, bladder and urethra). An organ can be normal or healthy, and alternatively, abnormal or unhealthy (e.g., cancerous).

An organ from a plant biological organism includes, for example, root, stem, leaf, flower, seed and fruit.

A tissue from a biological sample (e.g. an animal) includes a connective tissue, a muscle tissue, a nervous tissue, and an epithelial tissue. A tissue can be normal or healthy, and alternatively, abnormal or unhealthy (e.g., cancerous). A tissue from a biological sample (e.g. a plant) includes an epidermis, a vascular tissue and a ground tissue.

A cell can be prokaryotic or eukaryotic. A prokaryotic cell includes, for example, bacteria. A eukaryotic cell includes, for example, a fungus, a plant cell, and an animal cell. The types of an animal cell (e.g., a mammalian cell or a human cell) includes, for example, a cell from circulatory/immune system or organ (e.g., a B cell, a T cell (cytotoxic T cell, natural killer T cell, regulatory T cell, T helper cell), a natural killer cell, a granulocyte (e.g., basophil granulocyte, an eosinophil granulocyte, a neutrophil granulocyte and a hypersegmented neutrophil), a monocyte or macrophage, a red blood cell (e.g., reticulocyte), a mast cell, a thrombocyte or megakaryocyte, and a dendritic cell); a cell from an endocrine system or organ (e.g., a thyroid cell (e.g., thyroid epithelial cell, parafollicular cell), a parathyroid cell (e.g., parathyroid chief cell, oxyphil cell), an adrenal cell (e.g., chromaffin cell), and a pineal cell (e.g., pinealocyte)); a cell from a nervous system or organ (e.g., a glioblast (e.g., astrocyte and oligodendrocyte), a microglia, a magnocellular neurosecretory cell, a stellate cell, a boettcher cell, and a pituitary cell (e.g., gonadotrope, corticotrope, thyrotrope, somatotrope, and lactotroph)); a cell from a respiratory system or organ (e.g., a pneumocyte (a type I pneumocyte and a type II pneumocyte), a clara cell, a goblet cell, an alveolar macrophage); a cell from circular system or organ (e.g., myocardiocyte andpericyte); a cell from digestive system or organ (e.g., a gastric chief cell, a parietal cell, a goblet cell, a paneth cell, a G cell, a D cell, an ECL cell, an I cell, a K cell, an S cell, an enteroendocrine cell, an enterochromaffin cell, an APUD cell, a liver cell (e.g., a hepatocyte and Kupffer cell)); a cell from integumentary system or organ (e.g., a bone cell (e.g., an osteoblast, an osteocyte, and an osteoclast), a teeth cell (e.g., a cementoblast, and an ameloblast), a cartilage cell (e.g., a chondroblast and a chondrocyte), a skin/hair cell (e.g., a trichocyte, a keratinocyte, and a melanocyte (Nevus cell)), a muscle cell (e.g., myocyte), an adipocyte, a fibroblast, and a tendon cell), a cell from urinary system or organ (e.g., a podocyte, a juxtaglomerular cell, an intraglomerular mesangial cell, an extraglomerular mesangial cell, a kidney proximal tubule brush border cell, and a macula densa cell), and a cell from reproductive system or organ (e.g., a spermatozoon, a sertoli cell, a leydig cell, an ovum, an oocyte). A cell can be normal, healthy cell; or a diseased or unhealthy cell (e.g., a cancer cell).

A cell further includes a mammalian stem cell which include an embryonic stem cell, a fetal stem cell, an induced pluripotent stem cell, and an adult stem cell. A stem cell is a cell that is capable of undergoing cycles of cell division while maintaining an undifferentiated state and differentiating into specialized cell types. A stem cell can be an omnipotent stem cell, a pluripotent stem cell, a multipotent stem cell, an oligopotent stem cell and an unipotent stem cell (See, Hans R. Schöler (2007). "The Potential of Stem Cells: An Inventory" in Nikolaus Knoepffler, Dagmar Schipanski, and Stefan Lorenz Sorgner. Humanbiotechnology as Social Challenge. Ashgate Publishing, Ltd. pp. 28), any of which may be induced from a somatic cell. A stem cell may also include a cancer stem cell.

In another embodiment, "reprogramming a biological sample" used herein is exchangeable with or refers to modulating, altering, or changing the biological activities of the biological sample (e.g., cell) or modulating, altering, or changing the state or status of the biological sample from one to another. For example, by exposing a biological sample (e.g., a cell) to a transducible material, the biological activities of the cell (e.g., cell growth, cell division, cell metabolism, cell cycle, cell signaling, DNA replication, transcription, RNA splicing, protein synthesis, post-translation modification) are modulated or altered so as to lead to cell proliferation, differentiation (e.g., from progenitor cells to terminally differentiated cells), dedifferentiation (e.g., from terminally differentiated cells to pluripotent stem cells), transdifferentiation (e.g., from one type of terminally differentiated cells to another type of terminally differentiated cells), retrodifferentiation (e.g., from terminally differentiated cells to progenitor cells), transdertermination (e.g., from one type of progenitor cells to a type of terminally differentiated cells that are usually derived from another type of progenitor cells under natural conditions), apoptosis (e.g., cell death of cells or cancer cells), morphogenesis, and changes in the cell fate. For another example, the state of a biological sample can be altered or changed from abnormal or diseased state to normal or healthy state (e.g., from cancer cells to noncancer cells); from one cell type to another cell type (e.g., from undifferentiated stem cells to differentiated stem cells or specialized cells), from differentiated or specialized cells to undifferentiated cells or stem cells (e.g., an omnipotent stem cell, a pluripotent stem cell, a multipotent stem cell, an oligopotent stem cell and an unipotent stem cell)(e.g., from fibroblast cells to induced pluripotent stem cells (iPSCs)), from somatic cells to stem cells or induced stem cells, from one state of stem cells to another state of stem cells (e.g., from ominipotent stem cells to pluripotent stem cells), from one type of differentiated cells to another type of differentiated cells (e.g., T-cells to regulatory T cells, pancreatic exocrine cells to insulin-producing beta cells).

In another embodiment, a biological sample is exposed to a transducible material and reprogrammed. The biological sample can be exposed *in vitro, in vivo* or *ex vivo.* For example, the biological sample is exposed *in vitro* through contacting the sample with the transducible material in an environment outside of a living biological organism (e.g., in a cell culture system or a test tube). The biological sample is exposed *in vivo* through contacting the material with a biological organism containing the sample or introducing (e.g., through administration) the material into the organism. The transducible materials can be administered via any known administration route such as for example parenteral (e.g., subcutaneous, intraperitoneal, intravenous, including intravenous infusion, intramuscular, or intradermal injection) or non-parenteral (e.g., oral, intranasal, intraocular, sublingual, rectal, or topical) route. The biological sample is exposed *ex vivo* when the biological sample (e.g., a cell, a tissue or an organ) is taken outside the biological organism, contacted with the transducible material, and placed back to the same or different biological organisms. Examples of *ex vivo* exposures comprise removing a biological sample from the biological organism, exposing the biological sample to a transducible material, and transplanting the biological sample transduced with the transducible material back to the biological organism.

In certain embodiments, OG2-MEF cells are exposed to a composition comprising protein Oct4-11R, Sox2-11R, Klf4-11R and cMyc-11R and reprogrammed to induced pluripotent stem cells (iPSCs).

In certain embodiments, T cells are exposed to a composition comprising protein Foxp3-11R or His6-Foxp3-11R and programmed to regulatory T cells (Treg cells).

In certain embodiments liver and/or pancreatic exocrine cells are exposed to a composition comprising one or more proteins selected from the group consisting of Ngn3-11R, PDX1-11R, MafA-11R, NeuroD-11R, His6-Ngn3-11R, His6-PDX1-11R, His6-MafA-11R, and His6-NeuroD-11R and reprogrammed into insulin producing cells (e.g. β cells). In certain embodiments, the composition further comprises one or more adjuvant such as Islet growth factor (e.g. betacellulin). In certain embodiments, the composition comprises His6-Ngn3-11R, His6-PDX1-11R, and His6-MafA-11R. In certain embodiments, the composition comprises His6-Ngn3-11R, His6-PDX1-11R, His6-MafA-11R and betacellulin. Without bond to the mechanism, it is further contemplated that such reprogramming is through transdetermination and/or transdifferentiation.

Another aspect of the present disclosure relates to a method of treating, preventing or reducing a disease or condition in a biological organism by administering a composition comprising a transducible material into the organism. In certain embodiments, the composition is a pharmaceutical composition comprising a transducible material. In certain embodiments, the composition comprises a selective transducible material. The treatment, prevention or reduction of a disease or condition is associated with the change or reprogramming of a biological sample (e.g., a cell, a tissue or an organ) in the organism.

In certain embodiments, the disease or condition treatable by the method include, without limitations, tumor, cancer, metabolic diseases or conditions (e.g. type I and type II diabetes and obesity), inflammatory conditions, cardiac diseases, neurogenerative diseases (e.g. anemia, amyotrophic lateral sclerosis, spinal cord injury, bums, or arthritis), autoimmune diseases or conditions (e.g. acute disseminated encephalomyelitis (ADEM), Addison's disease, alopecia areata, ankylosing spondylitis, antiphospholipid antibody syndrome (APS), anemia (e.g. autoimmune hemolytic anemia and pernicious anaemia), arthritis, psoriatic arthritis, rheumatoid arthritis, diabetes mellitus type 1, autoimmune hepatitis, autoimmune inner ear disease, bullous pemphigoid, coeliac disease, Chagas disease, chronic obstructive pulmonary disease, Crohns disease, dermatomyositis, endometriosis, Goodpasture's syndrome, Graves' disease, Guillain-Barre syndrome (GBS), Hashimoto's disease, hidradenitis suppurativa, Kawasaki disease, IgA nephropathy, idiopathic thrombocytopenic purpura, interstitial cyctitis, lupus erythematosus, mixed connective tissue disease, morphea, multiple sclerosis (MS), myasthenia gravis, narcolepsy, neuromyotonia, pemphigus vulgaris, psoriasis, polymyositis, primary billiary cirrhosis, schizophrenia, scleroderma, Sjogren's syndrome, stiff person syndrome, temporal arteritis ("Giant cell arteritis"), ulcerative colitis, vasculitis, vitiligo, and Wegener's granulomatosis).

For example, it is contemplated that a transducible material can be administered to a biological organism having a tumor to activate the apoptosis of the tumor cells or make tumor cells more sensitive to chemotherapy, radiotherapy, or cancer drugs.

In certain embodiments, a transducible material can be administered to a biological organism to enhance or attenuate immune system and thus treat or prevent immune-related diseases or inflammatory diseases. For example, protein Foxp3-11R or His6-Foxp3-11R is transduced to T cells and programs them to Treg cells, which suppress the overactive immune system and thus is a treatment for auto-immune diseases

In certain embodiments, a transducible material can be administered to a biological organism to treat metabolic diseases or conditions such as type I diabetes, type II diabetes, or obesity. For example, to treat diabetes, a composition comprising a protein selected from the group consisting of Ngn3-11R, PDX1-11R, MafA-11R, NeuroD-11R, His6-Ngn3-11R, His6-PDX1-11R, His6-MafA-11R, His6-NeuroD-11R and any combination thereof can be transduced into liver and/or pancreatic exocrine cells and programs them to insulin producing cells (e.g. β cells). In certain embodiments, one or more adjuvant such as Islet growth factor (e.g. betacellulin) is/are also administered to the biological organism. In certain embodiments, the composition comprises His6-Ngn3-11R, His6-PDX1-11R, and His6-MafA-11R. In certain embodiments, the composition comprises His6-Ngn3-11R, His6-PDX1-11R, His6-MafA-11R and betacellulin. Without bond to the mechanism, it is further contemplated that such reprogramming is through transdetermination and/or transdifferentiation.

It is further contemplated that a transducible material can be administered to a biological organism to treat cardiac diseases such as myocardial infarction or ischemia.

Another aspect of the present disclosure relates to a method of reprogramming iPSCs, embryonic stem cells, or other types of stem or progenitor cells to certain types of somatic cells or progenitor cells, which can be developed as cell-based therapies for various diseases or conditions, including anemia, neurodegenerative diseases, cancer, amyotrophic lateral sclerosis, spinal cord injury, bums, heart diseases, diabetes, and arthritis. The stem cells or progenitor cells may be patient-specific or non-patient-specific, repaired to rid of molecular defects or not, before they are exposed to transducible materials for controlled differentiation or reprogramming. The reprogrammed cells may be enriched, purified, or manipulated before transplanted back to patients.

Another aspect of the present disclosure relates to a method of reprogramming iPSCs, embryonic stem cells, or other types of stem or progenitor cells to certain types of somatic cells or progenitor cells, which can be used as disease models for drug screening, mechanism study, toxicity assay, or other research and drug discovery and development tools. For example, the method comprises exposing an iPSC, an embryonic stem cell, or a progenitor cell to a composition comprising a transducible material to reprogram the iPSC, embryonic stem cell, or progenitor cell to a transplantable somatic cell or a transplantable progenitor cell; transplanting the transplantable somatic cell or transplantable progenitor cell into a biological sample or a biological organism; developing the biological sample or biological organism to become a disease model. For another example, the method comprises reprogramming patient-specific cells to iPSCs using a transducible materials; further generating different type of cells from patient specific iPSCs with or without tranducible materials; and developing a disease model using patient-specific iPSCs or iPSC-derived cells. For another example, the method of developing drug screening or toxicity models comprises reprogramming somatic cells, progenitor cells, or multipotent cells to iPSCs using a transducible material; further generating different type of cells from iPSCs with or without exposing to transducible materials; and using iPSCs and/or iPSC-derived cells to screen the effects and/or toxicities of different compounds.

Another aspect of the present disclosure relates to a method of developing cell-based therapies for various diseases or conditions comprising the step of reprogramming an iPSC, an embryonic stem cell, or a progenitor cell to a transplantable somatic or progenitor cell using a transducible material; transplanting the transplantable somatic or progenitor cell into a biological sample or biological organism; assessing the therapeutic effect of the transplantable somatic or progenitor cell.

Another aspect of the present disclosure relates to a method of identifying a effector domain, wherein the method comprises the steps of covalently linking a test effector domain to a know transduction domain to form a test transducible molecule; exposing the test molecule to a biological sample, and measuring the reprogramming of the biological sample to indicate whether the test effector domain can exerts a change in the biological sample. It is also contemplated that another aspect of the present disclosure relates to a method of identifying a transducible domain, wherein the method comprises the steps of covalently linking a known effector domain to a test transduction domain to form a test transducible molecule; exposing the test molecule to a biological sample, and measuring the location of the test molecule in or the reprogramming effect of the biological sample to indicate whether the test transduction domain can transduce the effector domain into the biological sample.

### EXAMPLES

The following examples are provided to better illustrate the claimed invention and are not to be interpreted in any way as limiting the scope of the invention. All specific compositions, materials, and methods described below, in whole or in part, fall within the scope of the invention. These specific compositions, materials, and methods are not intended to limit the invention, but merely to illustrate specific embodiments falling within the scope of the invention. One skilled in the art may develop equivalent compositions, materials, and methods without the exercise of inventive capacity and without departing from the scope of the invention. It will be understood that many variations can be made in the procedures herein described while still remaining within the bounds of the invention. It is the intention of the inventors that such variations are included within the scope of the invention.

Example 1. Reprogramming somatic cells to induced pluripotent stem cells (iPSCs)

1.a. Preparation of transducible material Oct4-11R, Sox2-11R, Klf4-11R, and cMyc-11R.

A poly-arginine protein transduction domain was fused to the C-terminal of each reprogramming proteins Oct4, Sox2, Klf4 and cMyc through a linker SEQ ID NO. 55 to form a fused protein Oct4-11R, Sox2-11R, Klf4-11R and cMyc-11R respectively (Figure 1A). These poly-arginine fused proteins were expressed in *E. Coli* in inclusion body form, which were then solubilized, refolded, and further purified to render transducible materials Oct4-11R, Sox2-11R, Klf4-11R and cMyc-11R. The protein identities were confirmed by mass spectrometry and Western blot analysis (Figure 1B).

1.b. Cell permeability and stability of transducible material Oct4-11R, Sox2-11R, Klf4-11R, and cMyc-11R

A transducible material (Oct4-11R, Sox2-11R, Klf4-11R, or cMyc-11R) was added to mouse embryonic fibroblast (MEF) cells at various concentrations for 6-72 hours. Cell morphology and protein presence were examined by immunocytochemistry. The transducible materials were found to enter cells at concentrations of 0.5-8 µg/ml within 6 hours, and translocated into nucleus (Figure 2). In addition, the transduced proteins were fairly stable inside of cells for up to 48 hours (Figure 3).

1.c. Reprogramming OG2/Oct4-GFP reporter MEF cells.

The protein transduction condition described in paragraph 0047 was used to reprogram OG2/Oct4-GFP reporter MEF cells. Cells were treated in 4 cycles. In each cycle the fibroblasts (initially seeded at the density of 5x10⁴ cells/well in a six-well plate) were first treated with transducible materials Oct4-11R, Sox2-11R, Klf4-11R and cMyc-11R at 8 µg/ml in the mESC growth media supplemented with or without 1 mM valproic acid (VPA, a inhibitor of the enzyme histone deacetylase 1 (HDAC1)) for overnight, followed by changing to the same media without the transducible material and VPA, and culturing for additional 36 hours before the next cycle of the treatment. After completing repeated protein transduction of a transducible material, the treated cells were transferred onto irradiated MEF feeder cells and kept in mESC growth media until colonies emerged around day 30-35 (Figure 4A). 3 GFP+ colonies per 5x10⁴ cells were obtained when the cells were transduced with Oct4-11R, Sox2-11R, Klf4-11R, and cMyc-11R and treated with VPA, and 1 GFP+ colony per 5x10⁴ cells were obtained when the cells were transduced with Oct4-11R, Sox2-11R, or Klf4-11R respectively and treated with VPA. Those initial GFP+ colonies were subsequently passaged under conventional mESC growth conditions to yield piPS cells, and were further characterized.

The generated murine piPS cells have been stably expanded for over twenty passages, and were morphologically indistinguishable to classic mES cells, forming compact domed small colonies (Figures 4B and 4C). They expressed typical pluripotency markers examined by immunocytochemistry and staining, including ALP (Figure 4D), Oct4, Nanog, Sox2, and SSEA1 (Figure 4E). RT-PCR analysis confirmed endogenous gene expression of these pluripotency markers and additional pluripotency genes (Figure 4F). A single cell survival assay also demonstrated that piPS cells clonally expanded efficiently as Oct4-positive colonies in feeder-free and N2/B27-chemically defined conditions. Furthermore, bisulphite genomic sequencing analyses of the Oct4 promoter revealed that it was demethylated in piPS cells similarly to the mES cells, while the MEFs' Oct4 promoter was hypermethylated (Figure 4G). This result further suggests a reactivation of the pluripotency transcription program in these piPS cells.

To examine the developmental potential of piPS cells, standard *in vitro* differentiation using embryoid bodies (EB) or monolayer chemically defined step-wise differentiation, as well as in vivo chimerism assays were performed. piPS cells efficiently formed EB in suspension, and differentiated into cells in the three primary germ layers, including primitive endoderm (AFP, Sox17), foregut endoderm (FoxA2), pancreatic cells endoderm (PDX1, Pax6), mesoderm (Brachyury), and neural (Sox1) and neuronal cells (βIII-tubulin)-ectoderm (Figures 5 and 6 A). These piPS cells efficiently incorporated into the inner cell mass of a blastocyst following aggregation with an 8-cell embryo, and led to chimerism with germline contribution (Figure 6B) *in vivo* after the aggregated embryos were transplanted into mice, as suggested by observation of Oct4-GFP+ cells in the gonad tissue in 2 out of 7 embryos (Figure 6B bottom). These *in vitro* and *in vivo* characterizations collectively confirm that the purified transduction material Oct4-11R, Sox2-11R, Klf4-11R, and cMyc-11R are able to reprogram MEFs to piPS cells, which are morphologically and functionally similar to conventional mES cells.

Example 2. Reprogramming of liver and pancreatic exocrine cells to insulin-producing beta cells by transducible materials His6-Ngn3-11R, His6-PDX1-11R and His6-MafA-11R in mouse.

A poly-arginine protein transduction domain was fused respectively to the C-terminal of each reprogramming protein (Ngn3, PDX1 and MafA) through a linker **(SEQ ID NO: 55)** to form His6-Ngn3-11R, His6-PDX1-11R and His6-MafA-11R respectively (Figure 7). His6 **(SEQ ID NO: 59)** was included to facilitate protein purification. These poly-arginine fused proteins were expressed in *E. Coli* in inclusion body form, which were then solubilized, refolded, and further purified to prepare transducible materials His6-Ngn3-11R, His6-PDX1-11R and His6-MafA-11R. The protein identities were confirmed by mass spectrometry and Western blot analysis.

Six CD-1 mice (Charles River Laboratory) were divided into two groups: the treatment group and the control group. Transducible material His6-Ngn3-11R (1mg/kg), His6-PDX1-11R (1mg/kg), and His6-MafA-11R (1mg/kg) were injected into each mouse by intraperitoneal (IP) in treatment group (Mouse-4, Mouse-5 and Mouse-6) and BSA (1mg/kg) was injected into each mouse in the control group (Mouse-1, Mouse-2 and Mouse-3). There was no Greenish-brown or Yellow aspirate when needle penetrated into each mouse peritonea. Injections were repeated every day for 7 days. Mice of both treatment and control group were sacrificed on the 3rd day after the completion of all injections. The mouse liver and pancreas were washed with 1X PBS and fixed by 4% paraformaldehyde for overnight. Then the liver and pancreatic tissues were processed by standard Paraffin *Embedding* protocol. The Tissue sections, 5-micro in thickness, were prepared routinely with histology microtomes and mounted on standard histology glass slides. The wax in tissues was dissolved by xylene during processing of tissue sections. Tissue sectioning and histologic and immunohistochemical staining were performed using routine methods. For indirect fluorescent-antibody (IFA) assay, the slides were blocked with 0.05% Tween-20 (TBST) and 3% BSA for 1 hour at RT and were incubated with mouse anti-insulin antibody (Invitrogen) at 4°C overnight. The slides were washed three times with PBS for 15 minutes at RT and incubated with fluorescein isothiocyanate (FITC) conjugated swine anti-mouse antibody (KPL) for 2 hours at RT. Same concentration of Mouse IgG was used as isotype control. Anti-DAPI antibody was added to slides as a nuclear marker. The slides were washed as before and mounted with aqueous mounting media (Biomeda, Foster City, CA). Endothelial markers were identified under the microscope (Olympus BX51, San Diego, CA) and merged cells were analyzed by Microsuite Biological Suite program (Olympus BX51, San Diego, CA) (Figures 8-11). The results showed that the treatment group had more insulin-producing cells (Figure 9) in livers comparing to the control group (Figure 8). The pancreas of the control group showed a cluster of insulin-producing cells (Figure 10), while the pancreas of the treatment group showed insulin-producing cells in bigger area (Figure 11). Therefore, the results showed that treatment of transducible materials His6-Ngn3-11R, His6-PDX1-11R, and His6-MafA-11R converted liver and/or pancreas cells to insulin-producing cells.

Example 3. Reprogramming of T cells and programs them to Treg cells using transducible material Foxp3.

A poly-arginine protein transduction domain was fused to the C-terminal of each reprogramming protein Foxp3 through a linker (SEQ ID NO: 55) to form His6-Foxp3-11R (Figure 7). His6 (SEQ ID NO: 59) was included to facilitate protein purification. The poly-arginine fused protein was expressed in E. *Coli* in inclusion body form, which were then solubilized, refolded, and further purified to prepare transducible materials His6-Foxp3-11R. The protein identities were confirmed by Western blot analysis.

100ml of healthy human blood was collected from a donor and the peripheral blood mononuclear cells (PBMCs) were isolated by density-gradient centrifugation using Histopaque-1077 (Sigma-Aldrich, St Louis, MO). CD14+ monocytes were removed by magnetic bead selection (Miltenyi Biotec, Auburn, CA).Briefly, 108 PBMCs were incubated with 200 µL anti-CD 14 microbeads (Miltenyi Biotec) in ice for 30 minutes. The cells were washed with cold 1X PBS with 2% FCS and centrifuged at 300g for 10 minutes and then resuspended in 1X PBS with 2% FCS. The cell suspension was applied to the magnetic column and unbinding cells were passed through by washing 3 times with 1X PBS with 2% FCS. The PBMC/mono- were harvested by centrifuged at 300g for 10 minutes.

The PBMC/mono- were cultured in 6-well plates (Becton Dickinson, Gaithersburg, MD) supplemented with 10% FBS, nonessential amino acids, 2 mM glutamine, 1 mM sodium pyruvate, 25 mM HEPES, 200 units/ml penicillin, and streptomycin at 37 °C and 5% CO₂. After 1 hour of culture, His6-Foxp3-11R (10µg/ml, 20 µg/ml, or 50 µg/ml) was added to the cells. BSA (100µg/ml) was added to another well as control. Same concentration of His6-Foxp3-11R or BSA was added after cultured for two days. After 5 days of culture, the cells were washed with PBS twice. The cells were re-suspended in 100 µL diluted and added rabbit anti-human CD25 for 90 minutes. The cells were washed three time with cold 1XPBS supplied 2% FBS and then the conjugated-PE mouse anti-human CD4 as well as conjugated-FITC goat anti-rabbit IgG were added to the cells for 60 minutes in ice. Conjugated-PE mouse IgG and rabbit IgG were incubated with another group cells as Isotype control. The cells were washed with PBS for flow cytometric analysis using a Beckman Coulter FC500 cytometer with Cytomics CXP software (Beckman Coulter, Fullerton, CA) (Figures 12 and 13). The results showed that the CD4+CD25+T cells (Treg cells) have dramatically increased with treatment of transducible material His6-Foxp3-11R, and the increase is protein-dose dependent.

100ml of healthy human blood was collected from a donor and the peripheral blood mononuclear cells (PBMCs) were isolated by density-gradient centrifugation using Histopaque-1077 (Sigma-Aldrich, St Louis, MO). The PBMC/mono⁻ were cultured in 6-well plates (Becton Dickinson, Gaithersburg, MD) supplemented with 10% FBS, nonessential amino acids, 2 mM glutamine, 1 mM sodium pyruvate, 25 mM HEPES, 200 units/ml penicillin, and streptomycin at 37°C and 5% CO2. After 1 hour of culture, Foxp3 (10µg/ml, 50 µg/ml, 100 µg/ml) were added to the cells. BSA (100µg/ml ) was added to another well as control. Same concentration of the Foxp3 or BSA was added after cultured two days. Following 5 days of culture, the cells were washed with PBS twice. The cells were re-suspended in 100 µL diluted and added rabbit anti-human CD25 for 90 minutes. The cells were washed three time with cold 1XPBS supplied 2% FBS and then the conjugated-PE mouse anti-human CD4 as well as conjugated-FITC goat anti-rabbit IgG were added to the cells for 60minutes in ice. Conjugated-PE mouse IgG and rabbit IgG were incubated with another group cells as Isotype control. The cells were washed with PBS for flow cytometric analysis using a Beckman Coulter FC500 cytometer with Cytomics CXP software (Beckman Coulter, Fullerton, CA) (Figures 14-17). The results showed that the CD4+CD25+T cells (Treg cells) have dramatically increased with treatment of transducible material His6-Foxp3-11R, and the increase is protein-dose dependent.

### Embodiments

1. A transducible material comprising an effector domain.
2. The transducible material of embodiment 1 further comprising a transduction domain.
3. The transducible material of embodiment 2, wherein the transduction domain is linked to the effector domain covalently, non-covalently or via a linker.
4. The transducible material of embodiment 1, wherein the effector domain is inherently transducible.
5. The transducible material of embodiment 1, wherein the effector domain is a polypeptide, a small molecule, or a polynucleotide.
6. The transducible material of embodiment 5 wherein the polypeptide is selected from the group consisting of Oct4, Sox2, Klf4, Lin28, Nanog, cMyc, Ngn3, PDX1, MafA, NeuroD, Foxp3, and any combination thereof.
7. The transducible material of embodiment 2, wherein the transduction domain is selected from the group consisting of a protein transduction domain, a cell penetrating peptide, a cell permeating peptide, an activatable cell penetrating peptide, a cell-targeting peptide and a polymer.
8. The transducible material of embodiment 7, wherein the protein transduction domain is selected from the group consisting of TAT, poly-arginine, Penetratin, Antennapedia, VP22, Transportan, MAP, MTS, PEP-1, Arg/Trp analogue, RRWRRWWRRWWRRW, polyguanidine peptoid, polyguanidine peptoid, inherent protein transduction domain, SEQ ID NO: 56, SEQ ID NO: 57, HIV-1 Rev, Flock house virus coat peptide, DNA-binding peptides, c-Fos, c-Jun and yeast GCN4.
9. The transducible material of embodiment 7, wherein the cell-targeting peptide is a peptides having an amino acid sequence selected from the group consisting of NGR, RGD, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 58.
10. The transducible material of embodiment 7, wherein the polymer is selected from the group consisting of a cationic lipid polymer and a nanoparticle.
11. The transducible material of embodiment 1, wherein the transducible material is capable of selectively transducing into one or more specific biological samples or capable of becoming transducible in a specific environment surrounding the biological sample.
12. The transducible material of embodiment 3, wherein the linker has an amino acid sequence set forth in SEQ ID: 55.
13. The transducible material of embodiment 1 further comprising one or more motifs that do not interrupt the function of the effector domain or the transduction domain.
14. The transducible material of embodiment 13 wherein the motif is covalently linked to the effector domain or the transduction domain.
15. The transducible material of embodiment 14, wherein the motif has an amino acid sequence set forth in SEQ ID: 59.
16. A method of reprogramming a biological sample, comprising:
   exposing the biological sample to at least one transducible material of embodiment 1.
17. The method of embodiment 16 wherein the biological material is a cell, a tissue, or an organ from a biological organism.
18. The method of embodiment 17 wherein the biological organism is a microorganism, a plant or an animal.
19. The method of embodiment 16 wherein the biological sample is reprogrammed so as to cause proliferation, differentiation, transdifferentiation, retrodifferentiation, transdertermination, dedifferentiation, apoptosis or morphogenesis.
20. The method of embodiment 17 wherein the cell is reprogrammed to change from a first type cell to a second type cell.
21. The method of embodiment 20 wherein the first type cell is a somatic cell and the second type cell is a stem cell.
22. The method of embodiment 21, wherein the transducible materials are Oct4-11R, Sox2-11R, Klf4-11R and cMyc-11R.
23. The method of embodiment 20, wherein the first type cell is a hepatocyte or a pancreatic cell and the second type cell is an insulin producing cell.
24. The method of embodiment 23, wherein the transducible materials are His-Ngn3-11R, His6-PDX1-11R and His6-MafA-11R.
25. The method of embodiment 20, wherein the first type cell is a T cell and the second type cell is a regulatory T cell.
26. The method of embodiment 25, wherein the transducible material is His6-Foxp3-11R.
27. The method of embodiment 20 wherein the first type cell is a stem cell or a first progenitor cell and the second type cell is a second progenitor cell or a somatic cell.
28. A pharmaceutical composition comprising a transducible material of embodiment 1.
29. The pharmaceutical composition of embodiment 28 further comprising one or more growth factors.
30. The pharmaceutical composition of embodiment 29, wherein the growth factor is an islet growth factor.
31. A composition comprising a biological sample and a transducible material of embodiment 1, wherein the transducible material has transduced into the biological sample.
32. A method of treating a disease or condition in a biological organism comprising administering a pharmaceutical composition comprising a transducible material of embodiment 1 into the biological organism.
33. The method of embodiment 32 wherein the disease or condition is selected from the group consisting of tumor, cancer, metabolic disease, inflammatory condition, cardiac disease, neurogenerative disease, and autoimmune disease.
34. The method of embodiment 33, wherein the metabolic diseases or conditions are diabetes.
35. The method of embodiment 33, wherein the neurogenerative disease is selected from the group consisting of Alzheimer's disease, amyotrophic lateral sclerosis (Lou Gehrig's Disease, Huntington's disease, and Parkingson's disease.
36. The method of embodiment 33, wherein the inflammatory disease is selected from the group consisting of anemia, spinal cord injury, bums, and arthritis.
37. The method of embodiment 33, wherein the autoimmune disease is selected from the group consisting of acute disseminated encephalomyelitis, Addison's disease, alopecia areata, ankylosing spondylitis, antiphospholipid antibody syndrome, anemia, autoimmune hemolytic anemia, pernicious anaemia, arthritis, psoriatic arthritis, rheumatoid arthritis, diabetes mellitus type 1, autoimmune hepatitis, autoimmune inner ear disease, bullous pemphigoid, coeliac disease, Chagas disease, chronic obstructive pulmonary disease, Crohns disease, dermatomyositis, endometriosis, Goodpasture's syndrome, Graves' disease, Guillain-Barre syndrome GBS, Hashimoto's disease, hidradenitis suppurativa, Kawasaki disease, IgA nephropathy, idiopathic thrombocytopenic purpura, interstitial cyctitis, lupus erythematosus, mixed connective tissue disease, morphea, multiple sclerosis, myasthenia gravis, narcolepsy, neuromyotonia, pemphigus vulgaris, psoriasis, polymyositis, primary billiary cirrhosis, schizophrenia, scleroderma, Sjogren's syndrome, stiff person syndrome, temporal arteritis, ulcerative colitis, vasculitis, vitiligo, and Wegener's granulomatosis.
38. A method of treating a disease or condition of a biological organism comprising:
   removing a biological sample from the biological organism;
   exposing the biological sample to a transducible material of embodiment 1; and
   transplanting the biological sample transduced with the transducible material back to the biological organism.
39. A method of developing cell-based therapies for various diseases or conditions comprising:
   reprogramming an iPSC, an embryonic stem cell, or a progenitor cell to a transplantable somatic cell or a transplantable progenitor cell by exposing the iPSC, the embryonic stem cell or the progenitor cell to at least one transducible material of embodiment 1;
   transplanting the transplantable somatic or progenitor cell into a biological sample or a biological organism; and
   assessing the therapeutic effects of the transplantable somatic or progenitor cell.
40. A method of developing a disease model comprising:
   exposing an iPSC, an embryonic stem cell, or a progenitor cell to at least one transducible material of embodiment 1 so as to reprogram into a transplantable somatic cell or a transplantable progenitor cell;
   transplanting the transplantable somatic or progenitor cell into a biological organism; and
   developing a disease model having the transplantable somatic or progenitor cell.
41. A method of a developing disease model comprising:
   exposing a patient-specific cell to at least one transducible material of embodiment 1 so as to reprogram into a patient specific iPSC ;
   generating a derived cell from the iPSC with or without the transducible materials; and
   developing a disease model using the patient-specific iPSC or iPSC-derived cell.
42. A method of identify an effector domain comprising:
   covalently linking a test effector domain to a transduction domain to form a test transducible molecule;
   exposing the test molecule to a biological sample, and
   measuring a reprogramming level of the biological sample.
43. A method of developing drug screening or toxicity models comprising:
   reprogramming a somatic cell, a progenitor cell, or a multipotent cell to an iPSC via exposing to at least one transducible material of embodiment 1;
   generating a derived cell from the iPSC with or without exposing to the transducible materials; and
   using the iPSC and/or the iPSC-derived cell to screen the effects and/or toxicities of different compounds.

## Claims

1. A method of reprogramming a biological sample, comprising:
exposing the biological sample to at least one transducible material comprising an effector domain, wherein optionally the biological sample is a cell, a tissue, or an organ from a biological organism, and wherein further, optionally, the biological organism is a microorganism, a plant or an animal.

2. The method of claim 1 wherein the biological sample is reprogrammed so as to cause transdifferentiation, transdertermination, proliferation, differentiation, retrodifferentiation, dedifferentiation, apoptosis or morphogenesis.

3. The method of claim 1 wherein the transducible material further comprises a transduction domain.

4. The method of claim 1, wherein the effector domain is a polypeptide, a small molecule, or a polynucleotide.

5. The method of claim 4, wherein the effector domain is selected from the group consisting of Pdx1, Ngn3, MafA, and NeuroD, and any combination thereof.

6. The method of claim 4, wherein the effector domain is:
(a) selected from the group consisting of Oct4, Sox2, Nanog, Klf4, Lin28 and c-Myc, and any combination thereof;
(b) Foxp3; or
(c) selected from the effector domains in (a) and (b) above and the effector domains of claim 5, and any combination thereof.

7. The method of claim 3, wherein the transduction domain is linked to the effector domain covalently, non-covalently or via a linker, and wherein optionally the linker has an amino acid sequence set forth in SEQ ID: 55.

8. The method of claim 1, wherein the effector domain is inherently transducible.

9. The method of claim 3, wherein the transduction domain is selected from the group consisting of a protein transduction domain, a cell penetrating peptide, a cell permeating peptide, an activatable cell penetrating peptide, a cell-targeting peptide and a polymer, optionally wherein:
(a) the protein transduction domain is selected from the group consisting of poly-arginine, TAT, Penetratin, Antennapedia, VP22, Transportan, MAP, MTS, Pep-1, Arg/Trp analogue, RRWRRWWRRWWRRW, polyguanidine peptoid, polyguanidine peptoid, inherent protein transduction domain, SEQ ID NO: 56, SEQ ID NO: 57, HIV-1 Rev, Flock house virus coat peptide, DNA-binding peptides, c-Fos, c-Jun and yeast GCN4;
(b) the cell-targeting peptide is a peptide having an amino acid sequence selected from the group consisting of NGR, RGD, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, and SEQ ID NO: 58; or
(c) the polymer is selected from the group consisting of a cationic lipid polymer and a nanoparticle.

10. The method of claim 1, wherein the transducible material is capable of selectively transducing into one or more specific biological samples or capable of becoming transducible in a specific environment surrounding the biological sample.

11. The method of claim 1, wherein the transducible material further comprises one or more motifs that do not interrupt the function of the effector domain or the transduction domain, wherein optionally the motif is covalently linked to the effector domain or the transduction domain, and wherein further, optionally, the motif has an amino acid sequence set forth in SEQ ID: 59.

12. The method of claim 1 wherein the cell is reprogrammed to change from a first type cell to a second type cell, wherein optionally:
(a) the first type cell is a hepatocyte or a pancreatic cell and the second type cell is an insulin producing cell, wherein optionally the transducible materials comprises an effector domain of selected from the group consisting of Pdx1, Ngn3, MafA, and NeuroD, and any combination thereof, optionally the transducible materials are PDX1-11R, Ngn3-11R, MafA-11R and NeuroD-11R;
(b) the first type cell is a somatic cell and the second type cell is a stem cell, wherein optionally the transducible materials comprises an effector domain of selected from the group consisting of Oct4, Sox2, Nanog, Klf4, Lin28 and c-Myc, and any combination thereof, optionally the transducible materials are Oct4-11R, Sox2-11R, Klf4-11R and cMyc-11R;
(c) the first type cell is a T cell and the second type cell is a regulatory T cell, wherein optionally the transducible material is Foxp3-11R; or
(d) the first type cell is a stem cell or a first progenitor cell and the second type cell is a second progenitor cell or a somatic cell.

13. A pharmaceutical composition comprising a transducible material comprising an effector domain selected from the group consisted of PDX1, Ngn3, MafA, and NeuroD, and any combination thereof, and further comprising a transduction domain, optionally further comprising one or more growth factors, wherein optionally the growth factor is an islet growth factor.

14. A composition comprising a biological sample and a transducible material comprising an effector domain selected from the group consisted of PDX1, Ngn3, MafA, and NeuroD, and any combination thereof, and further comprising a transduction domain, wherein the transducible material has transduced into the biological sample.

15. The pharmaceutical composition of claim 13 for use in treating a disease or condition in a biological organism.

16. The pharmaceutical composition according to claim 15 for the use of that claim, wherein the disease or condition is selected from the group consisting of tumor, cancer, and metabolic disease, wherein optionally the metabolic diseases or conditions are diabetes.

17. A biological sample removed from a biological organism and exposed to a transducible material comprising an effector domain selected from the group consisted of PDX1, Ngn3, MafA, and NeuroD, and any combination thereof, and further comprising a transduction domain, for use in transplantation back to the biological organism to treat a disease or condition of the biological organism.

18. A method of developing cell-based therapies for metabolic diseases or conditions comprising:
(i) reprogramming a hepatocyte or a pancreatic cell to a transplantable insulin producing cell by exposing the hepatocyte or the pancreatic cell to at least one transducible material comprising an effector domain selected from the group consisted of PDX1, Ngn3, MafA, and NeuroD, and any combination thereof, and further comprising a transduction domain; and
(ii) transplanting the transplantable insulin producing cell into a biological sample or a biological organism; and assessing the therapeutic effects of the transplantable insulin producing cell.


SEQUENCE LISTING

<110>  VIVOSCRIPT, INC.

<120>  COMPOSITIONS AND METHODS FOR RE-PROGRAMMING CELLS WITHOUT GENETIC
       MODIFICATION

<130>  P061166EP

<140>  EP_________._
<141>  2009-12-23

<150>  US 61/203,438
<151>  2008-12-23

<150>  US 61/210,586
<151>  2009-03-19

<150>  US 61/216,511
<151>  2009-05-18

<150>  US 61/226,659
<151>  2009-07-17

<160>  64

<170>  PatentIn version 3.5

<210>  1
<211>  360
<212>  PRT
<213>  Homo sapiens

<400>  1

Met Ala Gly His Leu Ala Ser Asp Phe Ala Phe Ser Pro Pro Pro Gly
1               5                   10                  15

Gly Gly Gly Asp Gly Pro Gly Gly Pro Glu Pro Gly Trp Val Asp Pro
            20                  25                  30

Arg Thr Trp Leu Ser Phe Gln Gly Pro Pro Gly Gly Pro Gly Ile Gly
        35                  40                  45

Pro Gly Val Gly Pro Gly Ser Glu Val Trp Gly Ile Pro Pro Cys Pro
    50                  55                  60

Pro Pro Tyr Glu Phe Cys Gly Gly Met Ala Tyr Cys Gly Pro Gln Val
65                  70                  75                  80

Gly Val Gly Leu Val Pro Gln Gly Gly Leu Glu Thr Ser Gln Pro Glu
                85                  90                  95

Gly Glu Ala Gly Val Gly Val Glu Ser Asn Ser Asp Gly Ala Ser Pro
            100                 105                 110

Glu Pro Cys Thr Val Thr Pro Gly Ala Val Lys Leu Glu Lys Glu Lys
        115                 120                 125

Leu Glu Gln Asn Pro Glu Glu Ser Gln Asp Ile Lys Ala Leu Gln Lys
    130                 135                 140

Glu Leu Glu Gln Phe Ala Lys Leu Leu Lys Gln Lys Arg Ile Thr Leu
145                 150                 155                 160

Gly Tyr Thr Gln Ala Asp Val Gly Leu Thr Leu Gly Val Leu Phe Gly
                165                 170                 175

Lys Val Phe Ser Gln Thr Thr Ile Cys Arg Phe Glu Ala Leu Gln Leu
            180                 185                 190

Ser Phe Lys Asn Met Cys Lys Leu Arg Pro Leu Leu Gln Lys Trp Val
        195                 200                 205

Glu Glu Ala Asp Asn Asn Glu Asn Leu Gln Glu Ile Cys Lys Ala Glu
    210                 215                 220

Thr Leu Val Gln Ala Arg Lys Arg Lys Arg Thr Ser Ile Glu Asn Arg
225                 230                 235                 240

Val Arg Gly Asn Leu Glu Asn Leu Phe Leu Gln Cys Pro Lys Pro Thr
                245                 250                 255

Leu Gln Gln Ile Ser His Ile Ala Gln Gln Leu Gly Leu Glu Lys Asp
            260                 265                 270

Val Val Arg Val Trp Phe Cys Asn Arg Arg Gln Lys Gly Lys Arg Ser
        275                 280                 285

Ser Ser Asp Tyr Ala Gln Arg Glu Asp Phe Glu Ala Ala Gly Ser Pro
    290                 295                 300

Phe Ser Gly Gly Pro Val Ser Phe Pro Leu Ala Pro Gly Pro His Phe
305                 310                 315                 320

Gly Thr Pro Gly Tyr Gly Ser Pro His Phe Thr Ala Leu Tyr Ser Ser
                325                 330                 335

Val Pro Phe Pro Glu Gly Glu Ala Phe Pro Pro Val Ser Val Thr Thr
            340                 345                 350

Leu Gly Ser Pro Met His Ser Asn
        355                 360

<210>  2
<211>  317
<212>  PRT
<213>  Homo sapiens

<400>  2

<210>  3
<211>  470
<212>  PRT
<213>  Homo sapiens

<400>  3

Met Ala Val Ser Asp Ala Leu Leu Pro Ser Phe Ser Thr Phe Ala Ser
1               5                   10                  15

Gly Pro Ala Gly Arg Glu Lys Thr Leu Arg Gln Ala Gly Ala Pro Asn
            20                  25                  30

Asn Arg Trp Arg Glu Glu Leu Ser His Met Lys Arg Leu Pro Pro Val
        35                  40                  45

Leu Pro Gly Arg Pro Tyr Asp Leu Ala Ala Ala Thr Val Ala Thr Asp
    50                  55                  60

Leu Glu Ser Gly Gly Ala Gly Ala Ala Cys Gly Gly Ser Asn Leu Ala
65                  70                  75                  80

Pro Leu Pro Arg Arg Glu Thr Glu Glu Phe Asn Asp Leu Leu Asp Leu
                85                  90                  95

Asp Phe Ile Leu Ser Asn Ser Leu Thr His Pro Pro Glu Ser Val Ala
            100                 105                 110

Ala Thr Val Ser Ser Ser Ala Ser Ala Ser Ser Ser Ser Ser Pro Ser
        115                 120                 125

Ser Ser Gly Pro Ala Ser Ala Pro Ser Thr Cys Ser Phe Thr Tyr Pro
    130                 135                 140

Ile Arg Ala Gly Asn Asp Pro Gly Val Ala Pro Gly Gly Thr Gly Gly
145                 150                 155                 160

Gly Leu Leu Tyr Gly Arg Glu Ser Ala Pro Pro Pro Thr Ala Pro Phe
                165                 170                 175

Asn Leu Ala Asp Ile Asn Asp Val Ser Pro Ser Gly Gly Phe Val Ala
            180                 185                 190

Glu Leu Leu Arg Pro Glu Leu Asp Pro Val Tyr Ile Pro Pro Gln Gln
        195                 200                 205

Pro Gln Pro Pro Gly Gly Gly Leu Met Gly Lys Phe Val Leu Lys Ala
    210                 215                 220

Ser Leu Ser Ala Pro Gly Ser Glu Tyr Gly Ser Pro Ser Val Ile Ser
225                 230                 235                 240

Val Ser Lys Gly Ser Pro Asp Gly Ser His Pro Val Val Val Ala Pro
                245                 250                 255

Tyr Asn Gly Gly Pro Pro Arg Thr Cys Pro Lys Ile Lys Gln Glu Ala
            260                 265                 270

Val Ser Ser Cys Thr His Leu Gly Ala Gly Pro Pro Leu Ser Asn Gly
        275                 280                 285

His Arg Pro Ala Ala His Asp Phe Pro Leu Gly Arg Gln Leu Pro Ser
    290                 295                 300

Arg Thr Thr Pro Thr Leu Gly Leu Glu Glu Val Leu Ser Ser Arg Asp
305                 310                 315                 320

Cys His Pro Ala Leu Pro Leu Pro Pro Gly Phe His Pro His Pro Gly
                325                 330                 335

Pro Asn Tyr Pro Ser Phe Leu Pro Asp Gln Met Gln Pro Gln Val Pro
            340                 345                 350

Pro Leu His Tyr Gln Glu Leu Met Pro Pro Gly Ser Cys Met Pro Glu
        355                 360                 365

Glu Pro Lys Pro Lys Arg Gly Arg Arg Ser Trp Pro Arg Lys Arg Thr
    370                 375                 380

Ala Thr His Thr Cys Asp Tyr Ala Gly Cys Gly Lys Thr Tyr Thr Lys
385                 390                 395                 400

Ser Ser His Leu Lys Ala His Leu Arg Thr His Thr Gly Glu Lys Pro
                405                 410                 415

Tyr His Cys Asp Trp Asp Gly Cys Gly Trp Lys Phe Ala Arg Ser Asp
            420                 425                 430

Glu Leu Thr Arg His Tyr Arg Lys His Thr Gly His Arg Pro Phe Gln
        435                 440                 445

Cys Gln Lys Cys Asp Arg Ala Phe Ser Arg Ser Asp His Leu Ala Leu
    450                 455                 460

His Met Lys Arg His Phe
465                 470

<210>  4
<211>  454
<212>  PRT
<213>  Homo sapiens

<400>  4

Met Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu

370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala
    450

<210>  5
<211>  209
<212>  PRT
<213>  Homo sapiens

<400>  5

Met Gly Ser Val Ser Asn Gln Gln Phe Ala Gly Gly Cys Ala Lys Ala
1               5                   10                  15

Ala Glu Glu Ala Pro Glu Glu Ala Pro Glu Asp Ala Ala Arg Ala Ala
            20                  25                  30

Asp Glu Pro Gln Leu Leu His Gly Ala Gly Ile Cys Lys Trp Phe Asn
        35                  40                  45

Val Arg Met Gly Phe Gly Phe Leu Ser Met Thr Ala Arg Ala Gly Val
    50                  55                  60

Ala Leu Asp Pro Pro Val Asp Val Phe Val His Gln Ser Lys Leu His
65                  70                  75                  80

Met Glu Gly Phe Arg Ser Leu Lys Glu Gly Glu Ala Val Glu Phe Thr
                85                  90                  95

Phe Lys Lys Ser Ala Lys Gly Leu Glu Ser Ile Arg Val Thr Gly Pro
            100                 105                 110

Gly Gly Val Phe Cys Ile Gly Ser Glu Arg Arg Pro Lys Gly Lys Ser
        115                 120                 125

Met Gln Lys Arg Arg Ser Lys Gly Asp Arg Cys Tyr Asn Cys Gly Gly

130                 135                 140

Leu Asp His His Ala Lys Glu Cys Lys Leu Pro Pro Gln Pro Lys Lys
145                 150                 155                 160

Cys His Phe Cys Gln Ser Ile Ser His Met Val Ala Ser Cys Pro Leu
                165                 170                 175

Lys Ala Gln Gln Gly Pro Ser Ala Gln Gly Lys Pro Thr Tyr Phe Arg
            180                 185                 190

Glu Glu Glu Glu Glu Ile His Ser Pro Thr Leu Leu Pro Glu Ala Gln
        195                 200                 205

Asn

<210>  6
<211>  305
<212>  PRT
<213>  Homo sapiens

<400>  6

Met Ser Val Asp Pro Ala Cys Pro Gln Ser Leu Pro Cys Phe Glu Ala
1               5                   10                  15

Ser Asp Cys Lys Glu Ser Ser Pro Met Pro Val Ile Cys Gly Pro Glu
            20                  25                  30

Glu Asn Tyr Pro Ser Leu Gln Met Ser Ser Ala Glu Met Pro His Thr
        35                  40                  45

Glu Thr Val Ser Pro Leu Pro Ser Ser Met Asp Leu Leu Ile Gln Asp
    50                  55                  60

Ser Pro Asp Ser Ser Thr Ser Pro Lys Gly Lys Gln Pro Thr Ser Ala
65                  70                  75                  80

Glu Asn Ser Val Ala Lys Lys Glu Asp Lys Val Pro Val Lys Lys Gln
                85                  90                  95

Lys Thr Arg Thr Val Phe Ser Ser Thr Gln Leu Cys Val Leu Asn Asp
            100                 105                 110

Arg Phe Gln Arg Gln Lys Tyr Leu Ser Leu Gln Gln Met Gln Glu Leu
        115                 120                 125

Ser Asn Ile Leu Asn Leu Ser Tyr Lys Gln Val Lys Thr Trp Phe Gln

130                 135                 140

Asn Gln Arg Met Lys Ser Lys Arg Trp Gln Lys Asn Asn Trp Pro Lys
145                 150                 155                 160

Asn Ser Asn Gly Val Thr Gln Lys Ala Ser Ala Pro Thr Tyr Pro Ser
                165                 170                 175

Leu Tyr Ser Ser Tyr His Gln Gly Cys Leu Val Asn Pro Thr Gly Asn
            180                 185                 190

Leu Pro Met Trp Ser Asn Gln Thr Trp Asn Asn Ser Thr Trp Ser Asn
        195                 200                 205

Gln Thr Gln Asn Ile Gln Ser Trp Ser Asn His Ser Trp Asn Thr Gln
    210                 215                 220

Thr Trp Cys Thr Gln Ser Trp Asn Asn Gln Ala Trp Asn Ser Pro Phe
225                 230                 235                 240

Tyr Asn Cys Gly Glu Glu Ser Leu Gln Ser Cys Met Gln Phe Gln Pro
                245                 250                 255

Asn Ser Pro Ala Ser Asp Leu Glu Ala Ala Leu Glu Ala Ala Gly Glu
            260                 265                 270

Gly Leu Asn Val Ile Gln Gln Thr Thr Arg Tyr Phe Ser Thr Pro Gln
        275                 280                 285

Thr Met Asp Leu Phe Leu Asn Tyr Ser Met Asn Met Gln Pro Glu Asp
    290                 295                 300

Val
305

<210>  7
<211>  356
<212>  PRT
<213>  Homo sapiens

<400>  7

Met Thr Lys Ser Tyr Ser Glu Ser Gly Leu Met Gly Glu Pro Gln Pro
1               5                   10                  15

Gln Gly Pro Pro Ser Trp Thr Asp Glu Cys Leu Ser Ser Gln Asp Glu
            20                  25                  30

Glu His Glu Ala Asp Lys Lys Glu Asp Asp Leu Glu Ala Met Asn Ala

35                  40                  45

Glu Glu Asp Ser Leu Arg Asn Gly Gly Glu Glu Glu Asp Glu Asp Glu
    50                  55                  60

Asp Leu Glu Glu Glu Glu Glu Glu Glu Glu Glu Asp Asp Asp Gln Lys
65                  70                  75                  80

Pro Lys Arg Arg Gly Pro Lys Lys Lys Lys Met Thr Lys Ala Arg Leu
                85                  90                  95

Glu Arg Phe Lys Leu Arg Arg Met Lys Ala Asn Ala Arg Glu Arg Asn
            100                 105                 110

Arg Met His Gly Leu Asn Ala Ala Leu Asp Asn Leu Arg Lys Val Val
        115                 120                 125

Pro Cys Tyr Ser Lys Thr Gln Lys Leu Ser Lys Ile Glu Thr Leu Arg
    130                 135                 140

Leu Ala Lys Asn Tyr Ile Trp Ala Leu Ser Glu Ile Leu Arg Ser Gly
145                 150                 155                 160

Lys Ser Pro Asp Leu Val Ser Phe Val Gln Thr Leu Cys Lys Gly Leu
                165                 170                 175

Ser Gln Pro Thr Thr Asn Leu Val Ala Gly Cys Leu Gln Leu Asn Pro
            180                 185                 190

Arg Thr Phe Leu Pro Glu Gln Asn Gln Asp Met Pro Pro His Leu Pro
        195                 200                 205

Thr Ala Ser Ala Ser Phe Pro Val His Pro Tyr Ser Tyr Gln Ser Pro
    210                 215                 220

Gly Leu Pro Ser Pro Pro Tyr Gly Thr Met Asp Ser Ser His Val Phe
225                 230                 235                 240

His Val Lys Pro Pro Pro His Ala Tyr Ser Ala Ala Leu Glu Pro Phe
                245                 250                 255

Phe Glu Ser Pro Leu Thr Asp Cys Thr Ser Pro Ser Phe Asp Gly Pro
            260                 265                 270

Leu Ser Pro Pro Leu Ser Ile Asn Gly Asn Phe Ser Phe Lys His Glu
        275                 280                 285

Pro Ser Ala Glu Phe Glu Lys Asn Tyr Ala Phe Thr Met His Tyr Pro
    290                 295                 300

Ala Ala Thr Leu Ala Gly Ala Gln Ser His Gly Ser Ile Phe Ser Gly
305                 310                 315                 320

Thr Ala Ala Pro Arg Cys Glu Ile Pro Ile Asp Asn Ile Met Ser Phe
                325                 330                 335

Asp Ser His Ser His His Glu Arg Val Met Ser Ala Gln Leu Asn Ala
            340                 345                 350

Ile Phe His Asp
        355

<210>  8
<211>  214
<212>  PRT
<213>  Homo sapiens

<400>  8

Met Thr Pro Gln Pro Ser Gly Ala Pro Thr Val Gln Val Thr Arg Glu
1               5                   10                  15

Thr Glu Arg Ser Phe Pro Arg Ala Ser Glu Asp Glu Val Thr Cys Pro
            20                  25                  30

Thr Ser Ala Pro Pro Ser Pro Thr Arg Thr Pro Gly Asn Cys Ala Glu
        35                  40                  45

Ala Glu Glu Gly Gly Cys Arg Gly Ala Pro Arg Lys Leu Arg Ala Arg
    50                  55                  60

Arg Gly Gly Arg Ser Arg Pro Lys Ser Glu Leu Ala Leu Ser Lys Gln
65                  70                  75                  80

Arg Arg Ser Arg Arg Lys Lys Ala Asn Asp Arg Glu Arg Asn Arg Met
                85                  90                  95

His Asp Leu Asn Ser Ala Leu Asp Ala Leu Arg Gly Val Leu Pro Thr
            100                 105                 110

Phe Pro Asp Asp Ala Lys Leu Thr Lys Ile Glu Thr Leu Arg Phe Ala
        115                 120                 125

His Asn Tyr Ile Trp Ala Leu Thr Gln Thr Leu Arg Ile Ala Asp His
    130                 135                 140

Ser Leu Tyr Ala Leu Glu Pro Pro Ala Pro His Cys Gly Glu Leu Gly
145                 150                 155                 160

Ser Pro Gly Gly Pro Pro Gly Asp Trp Gly Ser Leu Tyr Ser Pro Val
                165                 170                 175

Ser Gln Ala Gly Ser Leu Ser Pro Ala Ala Ser Leu Glu Glu Arg Pro
            180                 185                 190

Gly Leu Leu Gly Ala Thr Ser Ser Ala Cys Leu Ser Pro Gly Ser Leu
        195                 200                 205

Ala Phe Ser Asp Phe Leu
    210

<210>  9
<211>  283
<212>  PRT
<213>  Homo sapiens

<400>  9

<210>  10
<211>  352
<212>  PRT
<213>  Homo sapiens

<400>  10
   Met Ala Ala Glu Leu Ala Met Gly Ala Glu Leu Pro Ser Ser Pro Leu
   1               5                   10                  15
   Ala Ile Glu Tyr Val Asn Asp Phe Asp Leu Met Lys Phe Glu Val Lys
               20                  25                  30
   Lys Glu Pro Pro Glu Ala Glu Arg Phe Cys His Arg Leu Pro Pro Gly
           35                  40                  45
   Ser Leu Ser Ser Thr Pro Leu Ser Thr Pro Cys Ser Ser Val Pro Ser
       50                  55                  60
   Ser Pro Ser Phe Cys Ala Pro Ser Pro Gly Thr Gly Gly Gly Gly Gly
   65                  70                  75                  80
   Ala Gly Gly Gly Gly Gly Ser Ser Gln Ala Gly Gly Ala Pro Gly Pro
                   85                  90                  95
   Pro Ser Gly Gly Pro Gly Ala Val Gly Gly Thr Ser Gly Lys Pro Ala
               100                 105                 110
   Leu Glu Asp Leu Tyr Trp Met Ser Gly Tyr Gln His His Leu Asn Pro
           115                 120                 125
   Glu Ala Leu Asn Leu Thr Pro Glu Asp Ala Val Glu Ala Leu Ile Gly
       130                 135                 140
   Ser Gly His His Gly Ala His His Gly Ala His His Pro Ala Ala Ala
   145                 150                 155                 160
   Ala Ala Tyr Glu Ala Phe Arg Gly Pro Gly Phe Ala Gly Gly Gly Gly
                   165                 170                 175
   Ala Asp Asp Met Gly Ala Gly His His His Gly Ala His His Ala Ala
               180                 185                 190
   His His His His Ala Ala His His His His His His His His His Gly
           195                 200                 205
   Gly Ala Gly His Gly Gly Gly Ala Gly His His Val Arg Leu Glu Glu
       210                 215                 220
   Arg Phe Ser Asp Asp Gln Leu Val Ser Met Ser Val Arg Glu Leu Asn
   225                 230                 235                 240
   Arg Gln Leu Arg Gly Phe Ser Lys Glu Glu Val Ile Arg Leu Lys Gln
                   245                 250                 255
   Lys Arg Arg Thr Leu Lys Asn Arg Gly Tyr Ala Gln Ser Cys Arg Phe
               260                 265                 270
   Lys Arg Val Gln Gln Arg His Ile Leu Glu Ser Glu Lys Cys Gln Leu
           275                 280                 285
   Gln Ser Gln Val Glu Gln Leu Lys Leu Glu Val Gly Arg Leu Ala Lys
       290                 295                 300
   Glu Arg Asp Leu Tyr Lys Glu Lys Tyr Glu Lys Leu Ala Gly Arg Gly
   305                 310                 315                 320
   Gly Pro Gly Ser Ala Gly Gly Ala Gly Phe Pro Arg Glu Pro Ser Pro
                   325                 330                 335
   Pro Gln Ala Gly Pro Gly Gly Ala Lys Gly Thr Ala Asp Phe Phe Leu
               340                 345                 350

<210>  11
<211>  431
<212>  PRT
<213>  Homo sapiens

<400>  11

<210>  12
<211>  380
<212>  PRT
<213>  Artificial Sequence

<220>

<223>  transducible material

<400>  12

Met Ala Gly His Leu Ala Ser Asp Phe Ala Phe Ser Pro Pro Pro Gly
1               5                   10                  15

Gly Gly Gly Asp Gly Pro Gly Gly Pro Glu Pro Gly Trp Val Asp Pro
            20                  25                  30

Arg Thr Trp Leu Ser Phe Gln Gly Pro Pro Gly Gly Pro Gly Ile Gly
        35                  40                  45

Pro Gly Val Gly Pro Gly Ser Glu Val Trp Gly Ile Pro Pro Cys Pro
    50                  55                  60

Pro Pro Tyr Glu Phe Cys Gly Gly Met Ala Tyr Cys Gly Pro Gln Val
65                  70                  75                  80

Gly Val Gly Leu Val Pro Gln Gly Gly Leu Glu Thr Ser Gln Pro Glu
                85                  90                  95

Gly Glu Ala Gly Val Gly Val Glu Ser Asn Ser Asp Gly Ala Ser Pro
            100                 105                 110

Glu Pro Cys Thr Val Thr Pro Gly Ala Val Lys Leu Glu Lys Glu Lys
        115                 120                 125

Leu Glu Gln Asn Pro Glu Glu Ser Gln Asp Ile Lys Ala Leu Gln Lys
    130                 135                 140

Glu Leu Glu Gln Phe Ala Lys Leu Leu Lys Gln Lys Arg Ile Thr Leu
145                 150                 155                 160

Gly Tyr Thr Gln Ala Asp Val Gly Leu Thr Leu Gly Val Leu Phe Gly
                165                 170                 175

Lys Val Phe Ser Gln Thr Thr Ile Cys Arg Phe Glu Ala Leu Gln Leu
            180                 185                 190

Ser Phe Lys Asn Met Cys Lys Leu Arg Pro Leu Leu Gln Lys Trp Val
        195                 200                 205

Glu Glu Ala Asp Asn Asn Glu Asn Leu Gln Glu Ile Cys Lys Ala Glu
    210                 215                 220

Thr Leu Val Gln Ala Arg Lys Arg Lys Arg Thr Ser Ile Glu Asn Arg
225                 230                 235                 240

Val Arg Gly Asn Leu Glu Asn Leu Phe Leu Gln Cys Pro Lys Pro Thr
                245                 250                 255

Leu Gln Gln Ile Ser His Ile Ala Gln Gln Leu Gly Leu Glu Lys Asp
            260                 265                 270

Val Val Arg Val Trp Phe Cys Asn Arg Arg Gln Lys Gly Lys Arg Ser
        275                 280                 285

Ser Ser Asp Tyr Ala Gln Arg Glu Asp Phe Glu Ala Ala Gly Ser Pro
    290                 295                 300

Phe Ser Gly Gly Pro Val Ser Phe Pro Leu Ala Pro Gly Pro His Phe
305                 310                 315                 320

Gly Thr Pro Gly Tyr Gly Ser Pro His Phe Thr Ala Leu Tyr Ser Ser
                325                 330                 335

Val Pro Phe Pro Glu Gly Glu Ala Phe Pro Pro Val Ser Val Thr Thr
            340                 345                 350

Leu Gly Ser Pro Met His Ser Asn Glu Ser Gly Gly Gly Gly Ser Pro
        355                 360                 365

Gly Arg Arg Arg Arg Arg Arg Arg Arg Arg Arg Arg
    370                 375                 380

<210> 13
<211> 337
<212> PRT
<213> Artificial Sequence

<220>
<223> transducible material

<400> 13

Met Tyr Asn Met Met Glu Thr Glu Leu Lys Pro Pro Gly Pro Gln Gln
1               5                   10                  15

Thr Ser Gly Gly Gly Gly Gly Asn Ser Thr Ala Ala Ala Ala Gly Gly
            20                  25                  30

Asn Gln Lys Asn Ser Pro Asp Arg Val Lys Arg Pro Met Asn Ala Phe
        35                  40                  45

Met Val Trp Ser Arg Gly Gln Arg Arg Lys Met Ala Gln Glu Asn Pro
    50                  55                  60

Lys Met His Asn Ser Glu Ile Ser Lys Arg Leu Gly Ala Glu Trp Lys
65                  70                  75                  80

Leu Leu Ser Glu Thr Glu Lys Arg Pro Phe Ile Asp Glu Ala Lys Arg
                85                  90                  95

Leu Arg Ala Leu His Met Lys Glu His Pro Asp Tyr Lys Tyr Arg Pro
            100                 105                 110

Arg Arg Lys Thr Lys Thr Leu Met Lys Lys Asp Lys Tyr Thr Leu Pro
        115                 120                 125

Gly Gly Leu Leu Ala Pro Gly Gly Asn Ser Met Ala Ser Gly Val Gly
    130                 135                 140

Val Gly Ala Gly Leu Gly Ala Gly Val Asn Gln Arg Met Asp Ser Tyr
145                 150                 155                 160

Ala His Met Asn Gly Trp Ser Asn Gly Ser Tyr Ser Met Met Gln Asp
                165                 170                 175

Gln Leu Gly Tyr Pro Gln His Pro Gly Leu Asn Ala His Gly Ala Ala
            180                 185                 190

Gln Met Gln Pro Met His Arg Tyr Asp Val Ser Ala Leu Gln Tyr Asn
        195                 200                 205

Ser Met Thr Ser Ser Gln Thr Tyr Met Asn Gly Ser Pro Thr Tyr Ser
    210                 215                 220

Met Ser Tyr Ser Gln Gln Gly Thr Pro Gly Met Ala Leu Gly Ser Met
225                 230                 235                 240

Gly Ser Val Val Lys Ser Glu Ala Ser Ser Ser Pro Pro Val Val Thr
                245                 250                 255

Ser Ser Ser His Ser Arg Ala Pro Cys Gln Ala Gly Asp Leu Arg Asp
            260                 265                 270

Met Ile Ser Met Tyr Leu Pro Gly Ala Glu Val Pro Glu Pro Ala Ala
        275                 280                 285

Pro Ser Arg Leu His Met Ser Gln His Tyr Gln Ser Gly Pro Val Pro
    290                 295                 300

Gly Thr Ala Ile Asn Gly Thr Leu Pro Leu Ser His Met Glu Ser Gly
305                 310                 315                 320

Gly Gly Gly Ser Pro Gly Arg Arg Arg Arg Arg Arg Arg Arg Arg Arg
                325                 330                 335

Arg

<210>  14
<211>  490
<212>  PRT
<213>  Artificial Sequence

<220>
<223>  transducible material

<400>  14

Met Ala Val Ser Asp Ala Leu Leu Pro Ser Phe Ser Thr Phe Ala Ser
1               5                   10                  15

Gly Pro Ala Gly Arg Glu Lys Thr Leu Arg Gln Ala Gly Ala Pro Asn
            20                  25                  30

Asn Arg Trp Arg Glu Glu Leu Ser His Met Lys Arg Leu Pro Pro Val
        35                  40                  45

Leu Pro Gly Arg Pro Tyr Asp Leu Ala Ala Ala Thr Val Ala Thr Asp
    50                  55                  60

Leu Glu Ser Gly Gly Ala Gly Ala Ala Cys Gly Gly Ser Asn Leu Ala
65                  70                  75                  80

Pro Leu Pro Arg Arg Glu Thr Glu Glu Phe Asn Asp Leu Leu Asp Leu
                85                  90                  95

Asp Phe Ile Leu Ser Asn Ser Leu Thr His Pro Pro Glu Ser Val Ala
            100                 105                 110

Ala Thr Val Ser Ser Ser Ala Ser Ala Ser Ser Ser Ser Ser Pro Ser
        115                 120                 125

Ser Ser Gly Pro Ala Ser Ala Pro Ser Thr Cys Ser Phe Thr Tyr Pro
    130                 135                 140

Ile Arg Ala Gly Asn Asp Pro Gly Val Ala Pro Gly Gly Thr Gly Gly
145                 150                 155                 160

Gly Leu Leu Tyr Gly Arg Glu Ser Ala Pro Pro Pro Thr Ala Pro Phe
                165                 170                 175

Asn Leu Ala Asp Ile Asn Asp Val Ser Pro Ser Gly Gly Phe Val Ala
        180                 185                 190

Glu Leu Leu Arg Pro Glu Leu Asp Pro Val Tyr Ile Pro Pro Gln Gln
    195                 200                 205

Pro Gln Pro Pro Gly Gly Gly Leu Met Gly Lys Phe Val Leu Lys Ala
    210                 215                 220

Ser Leu Ser Ala Pro Gly Ser Glu Tyr Gly Ser Pro Ser Val Ile Ser
225                 230                 235                 240

Val Ser Lys Gly Ser Pro Asp Gly Ser His Pro Val Val Val Ala Pro
                245                 250                 255

Tyr Asn Gly Gly Pro Pro Arg Thr Cys Pro Lys Ile Lys Gln Glu Ala
            260                 265                 270

Val Ser Ser Cys Thr His Leu Gly Ala Gly Pro Pro Leu Ser Asn Gly
        275                 280                 285

His Arg Pro Ala Ala His Asp Phe Pro Leu Gly Arg Gln Leu Pro Ser
    290                 295                 300

Arg Thr Thr Pro Thr Leu Gly Leu Glu Glu Val Leu Ser Ser Arg Asp
305                 310                 315                 320

Cys His Pro Ala Leu Pro Leu Pro Pro Gly Phe His Pro His Pro Gly
                325                 330                 335

Pro Asn Tyr Pro Ser Phe Leu Pro Asp Gln Met Gln Pro Gln Val Pro
            340                 345                 350

Pro Leu His Tyr Gln Glu Leu Met Pro Pro Gly Ser Cys Met Pro Glu
        355                 360                 365

Glu Pro Lys Pro Lys Arg Gly Arg Arg Ser Trp Pro Arg Lys Arg Thr
    370                 375                 380

Ala Thr His Thr Cys Asp Tyr Ala Gly Cys Gly Lys Thr Tyr Thr Lys
385                 390                 395                 400

Ser Ser His Leu Lys Ala His Leu Arg Thr His Thr Gly Glu Lys Pro
                405                 410                 415

Tyr His Cys Asp Trp Asp Gly Cys Gly Trp Lys Phe Ala Arg Ser Asp
            420                 425                 430

Glu Leu Thr Arg His Tyr Arg Lys His Thr Gly His Arg Pro Phe Gln
        435                 440                 445

Cys Gln Lys Cys Asp Arg Ala Phe Ser Arg Ser Asp His Leu Ala Leu
    450                 455                 460

His Met Lys Arg His Phe Glu Ser Gly Gly Gly Gly Ser Pro Gly Arg
465                 470                 475                 480

Arg Arg Arg Arg Arg Arg Arg Arg Arg Arg
                485                 490

<210>  15
<211>  474
<212>  PRT
<213>  Artificial Sequence

<220>
<223>  transducible material

<400>  15

Met Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala Glu Ser Gly Gly Gly Gly Ser Pro Gly Arg
    450                 455                 460

Arg Arg Arg Arg Arg Arg Arg Arg Arg Arg
465                 470

<210> 16
<211> 229
<212> PRT
<213> Artificial Sequence

<220>
<223> transducible material

<400> 16

Met Gly Ser Val Ser Asn Gln Gln Phe Ala Gly Gly Cys Ala Lys Ala
1               5                   10                  15

Ala Glu Glu Ala Pro Glu Glu Ala Pro Glu Asp Ala Ala Arg Ala Ala
            20                  25                  30

Asp Glu Pro Gln Leu Leu His Gly Ala Gly Ile Cys Lys Trp Phe Asn
        35                  40                  45

Val Arg Met Gly Phe Gly Phe Leu Ser Met Thr Ala Arg Ala Gly Val
    50                  55                  60

Ala Leu Asp Pro Pro Val Asp Val Phe Val His Gln Ser Lys Leu His
65                  70                  75                  80

Met Glu Gly Phe Arg Ser Leu Lys Glu Gly Glu Ala Val Glu Phe Thr
                85                  90                  95

Phe Lys Lys Ser Ala Lys Gly Leu Glu Ser Ile Arg Val Thr Gly Pro
            100                 105                 110

Gly Gly Val Phe Cys Ile Gly Ser Glu Arg Arg Pro Lys Gly Lys Ser
        115                 120                 125

Met Gln Lys Arg Arg Ser Lys Gly Asp Arg Cys Tyr Asn Cys Gly Gly
    130                 135                 140

Leu Asp His His Ala Lys Glu Cys Lys Leu Pro Pro Gln Pro Lys Lys
145                 150                 155                 160

Cys His Phe Cys Gln Ser Ile Ser His Met Val Ala Ser Cys Pro Leu
                165                 170                 175

Lys Ala Gln Gln Gly Pro Ser Ala Gln Gly Lys Pro Thr Tyr Phe Arg
            180                 185                 190

Glu Glu Glu Glu Glu Ile His Ser Pro Thr Leu Leu Pro Glu Ala Gln
        195                 200                 205

Asn Glu Ser Gly Gly Gly Gly Ser Pro Gly Arg Arg Arg Arg Arg Arg
    210                 215                 220

Arg Arg Arg Arg Arg
225

<210>  17
<211>  325
<212>  PRT
<213>  Artificial Sequence

<220>
<223>  transducible material

<400>  17

Met Ser Val Asp Pro Ala Cys Pro Gln Ser Leu Pro Cys Phe Glu Ala
1               5                   10                  15

Ser Asp Cys Lys Glu Ser Ser Pro Met Pro Val Ile Cys Gly Pro Glu
            20                  25                  30

Glu Asn Tyr Pro Ser Leu Gln Met Ser Ser Ala Glu Met Pro His Thr
        35                  40                  45

Glu Thr Val Ser Pro Leu Pro Ser Ser Met Asp Leu Leu Ile Gln Asp
    50                  55                  60

Ser Pro Asp Ser Ser Thr Ser Pro Lys Gly Lys Gln Pro Thr Ser Ala
65                  70                  75                  80

Glu Asn Ser Val Ala Lys Lys Glu Asp Lys Val Pro Val Lys Lys Gln
                85                  90                  95

Lys Thr Arg Thr Val Phe Ser Ser Thr Gln Leu Cys Val Leu Asn Asp

            100                 105                 110

Arg Phe Gln Arg Gln Lys Tyr Leu Ser Leu Gln Gln Met Gln Glu Leu
        115                 120                 125

Ser Asn Ile Leu Asn Leu Ser Tyr Lys Gln Val Lys Thr Trp Phe Gln
    130                 135                 140

Asn Gln Arg Met Lys Ser Lys Arg Trp Gln Lys Asn Asn Trp Pro Lys
145                 150                 155                 160

Asn Ser Asn Gly Val Thr Gln Lys Ala Ser Ala Pro Thr Tyr Pro Ser
                165                 170                 175

Leu Tyr Ser Ser Tyr His Gln Gly Cys Leu Val Asn Pro Thr Gly Asn
            180                 185                 190

Leu Pro Met Trp Ser Asn Gln Thr Trp Asn Asn Ser Thr Trp Ser Asn
        195                 200                 205

Gln Thr Gln Asn Ile Gln Ser Trp Ser Asn His Ser Trp Asn Thr Gln
    210                 215                 220

Thr Trp Cys Thr Gln Ser Trp Asn Asn Gln Ala Trp Asn Ser Pro Phe
225                 230                 235                 240

Tyr Asn Cys Gly Glu Glu Ser Leu Gln Ser Cys Met Gln Phe Gln Pro
                245                 250                 255

Asn Ser Pro Ala Ser Asp Leu Glu Ala Ala Leu Glu Ala Ala Gly Glu
            260                 265                 270

Gly Leu Asn Val Ile Gln Gln Thr Thr Arg Tyr Phe Ser Thr Pro Gln
        275                 280                 285

Thr Met Asp Leu Phe Leu Asn Tyr Ser Met Asn Met Gln Pro Glu Asp
    290                 295                 300

Val Glu Ser Gly Gly Gly Gly Ser Pro Gly Arg Arg Arg Arg Arg Arg
305                 310                 315                 320

Arg Arg Arg Arg Arg
                325

<210> 18
<211> 376
<212> PRT
<213> Artificial Sequence

<220>
<223>  transducible material

<400>  18

Met Thr Lys Ser Tyr Ser Glu Ser Gly Leu Met Gly Glu Pro Gln Pro
1               5                   10                  15

Gln Gly Pro Pro Ser Trp Thr Asp Glu Cys Leu Ser Ser Gln Asp Glu
            20                  25                  30

Glu His Glu Ala Asp Lys Lys Glu Asp Asp Leu Glu Ala Met Asn Ala
        35                  40                  45

Glu Glu Asp Ser Leu Arg Asn Gly Gly Glu Glu Glu Asp Glu Asp Glu
    50                  55                  60

Asp Leu Glu Glu Glu Glu Glu Glu Glu Glu Glu Asp Asp Asp Gln Lys
65                  70                  75                  80

Pro Lys Arg Arg Gly Pro Lys Lys Lys Lys Met Thr Lys Ala Arg Leu
                85                  90                  95

Glu Arg Phe Lys Leu Arg Arg Met Lys Ala Asn Ala Arg Glu Arg Asn
            100                 105                 110

Arg Met His Gly Leu Asn Ala Ala Leu Asp Asn Leu Arg Lys Val Val
        115                 120                 125

Pro Cys Tyr Ser Lys Thr Gln Lys Leu Ser Lys Ile Glu Thr Leu Arg
    130                 135                 140

Leu Ala Lys Asn Tyr Ile Trp Ala Leu Ser Glu Ile Leu Arg Ser Gly
145                 150                 155                 160

Lys Ser Pro Asp Leu Val Ser Phe Val Gln Thr Leu Cys Lys Gly Leu
                165                 170                 175

Ser Gln Pro Thr Thr Asn Leu Val Ala Gly Cys Leu Gln Leu Asn Pro
            180                 185                 190

Arg Thr Phe Leu Pro Glu Gln Asn Gln Asp Met Pro Pro His Leu Pro
        195                 200                 205

Thr Ala Ser Ala Ser Phe Pro Val His Pro Tyr Ser Tyr Gln Ser Pro
    210                 215                 220

Gly Leu Pro Ser Pro Pro Tyr Gly Thr Met Asp Ser Ser His Val Phe

225                 230                 235                 240

His Val Lys Pro Pro Pro His Ala Tyr Ser Ala Ala Leu Glu Pro Phe
                245                 250                 255

Phe Glu Ser Pro Leu Thr Asp Cys Thr Ser Pro Ser Phe Asp Gly Pro
            260                 265                 270

Leu Ser Pro Pro Leu Ser Ile Asn Gly Asn Phe Ser Phe Lys His Glu
        275                 280                 285

Pro Ser Ala Glu Phe Glu Lys Asn Tyr Ala Phe Thr Met His Tyr Pro
    290                 295                 300

Ala Ala Thr Leu Ala Gly Ala Gln Ser His Gly Ser Ile Phe Ser Gly
305                 310                 315                 320

Thr Ala Ala Pro Arg Cys Glu Ile Pro Ile Asp Asn Ile Met Ser Phe
                325                 330                 335

Asp Ser His Ser His His Glu Arg Val Met Ser Ala Gln Leu Asn Ala
            340                 345                 350

Ile Phe His Asp Glu Ser Gly Gly Gly Gly Ser Pro Gly Arg Arg Arg
        355                 360                 365

Arg Arg Arg Arg Arg Arg Arg Arg
    370                 375

<210>  19
<211>  234
<212>  PRT
<213>  Artificial Sequence

<220>
<223>  transducible material

<400>  19

Met Thr Pro Gln Pro Ser Gly Ala Pro Thr Val Gln Val Thr Arg Glu
1               5                   10                  15

Thr Glu Arg Ser Phe Pro Arg Ala Ser Glu Asp Glu Val Thr Cys Pro
            20                  25                  30

Thr Ser Ala Pro Pro Ser Pro Thr Arg Thr Pro Gly Asn Cys Ala Glu
        35                  40                  45

Ala Glu Glu Gly Gly Cys Arg Gly Ala Pro Arg Lys Leu Arg Ala Arg
    50                  55                  60

Arg Gly Gly Arg Ser Arg Pro Lys Ser Glu Leu Ala Leu Ser Lys Gln
65                  70                  75                  80

Arg Arg Ser Arg Arg Lys Lys Ala Asn Asp Arg Glu Arg Asn Arg Met
                85                  90                  95

His Asp Leu Asn Ser Ala Leu Asp Ala Leu Arg Gly Val Leu Pro Thr
            100                 105                 110

Phe Pro Asp Asp Ala Lys Leu Thr Lys Ile Glu Thr Leu Arg Phe Ala
        115                 120                 125

His Asn Tyr Ile Trp Ala Leu Thr Gln Thr Leu Arg Ile Ala Asp His
    130                 135                 140

Ser Leu Tyr Ala Leu Glu Pro Pro Ala Pro His Cys Gly Glu Leu Gly
145                 150                 155                 160

Ser Pro Gly Gly Pro Pro Gly Asp Trp Gly Ser Leu Tyr Ser Pro Val
                165                 170                 175

Ser Gln Ala Gly Ser Leu Ser Pro Ala Ala Ser Leu Glu Glu Arg Pro
            180                 185                 190

Gly Leu Leu Gly Ala Thr Ser Ser Ala Cys Leu Ser Pro Gly Ser Leu
        195                 200                 205

Ala Phe Ser Asp Phe Leu Glu Ser Gly Gly Gly Gly Ser Pro Gly Arg
    210                 215                 220

Arg Arg Arg Arg Arg Arg Arg Arg Arg Arg
225                 230

<210>  20
<211>  303
<212>  PRT
<213>  Artificial Sequence

<220>
<223>  transducible material

<400>  20
   Met Asn Gly Glu Glu Gln Tyr Tyr Ala Ala Thr Gln Leu Tyr Lys Asp
   1               5                   10                  15
   Pro Cys Ala Phe Gln Arg Gly Pro Ala Pro Glu Phe Ser Ala Ser Pro
               20                  25                  30
   Pro Ala Cys Leu Tyr Met Gly Arg Gln Pro Pro Pro Pro Pro Pro His
           35                  40                  45
   Pro Phe Pro Gly Ala Leu Gly Ala Leu Glu Gln Gly Ser Pro Pro Asp
       50                  55                  60
   Ile Ser Pro Tyr Glu Val Pro Pro Leu Ala Asp Asp Pro Ala Val Ala
   65                  70                  75                  80
   His Leu His His His Leu Pro Ala Gln Leu Ala Leu Pro His Pro Pro
                   85                  90                  95
   Ala Gly Pro Phe Pro Glu Gly Ala Glu Pro Gly Val Leu Glu Glu Pro
               100                 105                 110
   Asn Arg Val Gln Leu Pro Phe Pro Trp Met Lys Ser Thr Lys Ala His
           115                 120                 125
   Ala Trp Lys Gly Gln Trp Ala Gly Gly Ala Tyr Ala Ala Glu Pro Glu
       130                 135                 140
   Glu Asn Lys Arg Thr Arg Thr Ala Tyr Thr Arg Ala Gln Leu Leu Glu
   145                 150                 155                 160
   Leu Glu Lys Glu Phe Leu Phe Asn Lys Tyr Ile Ser Arg Pro Arg Arg
                   165                 170                 175
   Val Glu Leu Ala Val Met Leu Asn Leu Thr Glu Arg His Ile Lys Ile
               180                 185                 190
   Trp Phe Gln Asn Arg Arg Met Lys Trp Lys Lys Glu Glu Asp Lys Lys
           195                 200                 205
   Arg Gly Gly Gly Thr Ala Val Gly Gly Gly Gly Val Ala Glu Pro Glu
       210                 215                 220
   Gln Asp Cys Ala Val Thr Ser Gly Glu Glu Leu Leu Ala Leu Pro Pro
   225                 230                 235                 240
   Pro Pro Pro Pro Gly Gly Ala Val Pro Pro Ala Ala Pro Val Ala Ala
                   245                 250                 255
   Arg Glu Gly Arg Leu Pro Pro Gly Leu Ser Ala Ser Pro Gln Pro Ser
               260                 265                 270
   Ser Val Ala Pro Arg Arg Pro Gln Glu Pro Arg Glu Ser Gly Gly Gly
           275                 280                 285
   Gly Ser Pro Gly Arg Arg Arg Arg Arg Arg Arg Arg Arg Arg Arg
       290                 295                 300

<210>  21
<211>  372
<212>  PRT
<213>  Artificial Sequence

<220>
<223>  transducible material

<400>  21

Met Ala Ala Glu Leu Ala Met Gly Ala Glu Leu Pro Ser Ser Pro Leu
1               5                   10                  15

Ala Ile Glu Tyr Val Asn Asp Phe Asp Leu Met Lys Phe Glu Val Lys
            20                  25                  30

Lys Glu Pro Pro Glu Ala Glu Arg Phe Cys His Arg Leu Pro Pro Gly
        35                  40                  45

Ser Leu Ser Ser Thr Pro Leu Ser Thr Pro Cys Ser Ser Val Pro Ser
    50                  55                  60

Ser Pro Ser Phe Cys Ala Pro Ser Pro Gly Thr Gly Gly Gly Gly Gly
65                  70                  75                  80

Ala Gly Gly Gly Gly Gly Ser Ser Gln Ala Gly Gly Ala Pro Gly Pro
                85                  90                  95

Pro Ser Gly Gly Pro Gly Ala Val Gly Gly Thr Ser Gly Lys Pro Ala
            100                 105                 110

Leu Glu Asp Leu Tyr Trp Met Ser Gly Tyr Gln His His Leu Asn Pro
        115                 120                 125

Glu Ala Leu Asn Leu Thr Pro Glu Asp Ala Val Glu Ala Leu Ile Gly
    130                 135                 140

Ser Gly His His Gly Ala His His Gly Ala His His Pro Ala Ala Ala
145                 150                 155                 160

Ala Ala Tyr Glu Ala Phe Arg Gly Pro Gly Phe Ala Gly Gly Gly Gly
                165                 170                 175

Ala Asp Asp Met Gly Ala Gly His His His Gly Ala His His Ala Ala
            180                 185                 190

His His His His Ala Ala His His His His His His His His His Gly
        195                 200                 205

Gly Ala Gly His Gly Gly Gly Ala Gly His His Val Arg Leu Glu Glu
    210                 215                 220

Arg Phe Ser Asp Asp Gln Leu Val Ser Met Ser Val Arg Glu Leu Asn
225                 230                 235                 240

Arg Gln Leu Arg Gly Phe Ser Lys Glu Glu Val Ile Arg Leu Lys Gln
                245                 250                 255

Lys Arg Arg Thr Leu Lys Asn Arg Gly Tyr Ala Gln Ser Cys Arg Phe
            260                 265                 270

Lys Arg Val Gln Gln Arg His Ile Leu Glu Ser Glu Lys Cys Gln Leu
        275                 280                 285

Gln Ser Gln Val Glu Gln Leu Lys Leu Glu Val Gly Arg Leu Ala Lys
    290                 295                 300

Glu Arg Asp Leu Tyr Lys Glu Lys Tyr Glu Lys Leu Ala Gly Arg Gly
305                 310                 315                 320

Gly Pro Gly Ser Ala Gly Gly Ala Gly Phe Pro Arg Glu Pro Ser Pro
                325                 330                 335

Pro Gln Ala Gly Pro Gly Gly Ala Lys Gly Thr Ala Asp Phe Phe Leu
            340                 345                 350

Glu Ser Gly Gly Gly Gly Ser Pro Gly Arg Arg Arg Arg Arg Arg Arg
        355                 360                 365

Arg Arg Arg Arg
    370

<210>  22
<211>  451
<212>  PRT
<213>  Artificial Sequence

<220>
<223>  transducible material

<400>  22

Met Pro Asn Pro Arg Pro Gly Lys Pro Ser Ala Pro Ser Leu Ala Leu
1               5                   10                  15

Gly Pro Ser Pro Gly Ala Ser Pro Ser Trp Arg Ala Ala Pro Lys Ala
            20                  25                  30

Ser Asp Leu Leu Gly Ala Arg Gly Pro Gly Gly Thr Phe Gln Gly Arg
        35                  40                  45

Asp Leu Arg Gly Gly Ala His Ala Ser Ser Ser Ser Leu Asn Pro Met
    50                  55                  60

Pro Pro Ser Gln Leu Gln Leu Pro Thr Leu Pro Leu Val Met Val Ala
65                  70                  75                  80

Pro Ser Gly Ala Arg Leu Gly Pro Leu Pro His Leu Gln Ala Leu Leu
                85                  90                  95

Gln Asp Arg Pro His Phe Met His Gln Leu Ser Thr Val Asp Ala His
            100                 105                 110

Ala Arg Thr Pro Val Leu Gln Val His Pro Leu Glu Ser Pro Ala Met
        115                 120                 125

Ile Ser Leu Thr Pro Pro Thr Thr Ala Thr Gly Val Phe Ser Leu Lys
    130                 135                 140

Ala Arg Pro Gly Leu Pro Pro Gly Ile Asn Val Ala Ser Leu Glu Trp
145                 150                 155                 160

Val Ser Arg Glu Pro Ala Leu Leu Cys Thr Phe Pro Asn Pro Ser Ala
                165                 170                 175

Pro Arg Lys Asp Ser Thr Leu Ser Ala Val Pro Gln Ser Ser Tyr Pro
            180                 185                 190

Leu Leu Ala Asn Gly Val Cys Lys Trp Pro Gly Cys Glu Lys Val Phe
        195                 200                 205

Glu Glu Pro Glu Asp Phe Leu Lys His Cys Gln Ala Asp His Leu Leu
    210                 215                 220

Asp Glu Lys Gly Arg Ala Gln Cys Leu Leu Gln Arg Glu Met Val Gln
225                 230                 235                 240

Ser Leu Glu Gln Gln Leu Val Leu Glu Lys Glu Lys Leu Ser Ala Met
                245                 250                 255

Gln Ala His Leu Ala Gly Lys Met Ala Leu Thr Lys Ala Ser Ser Val
            260                 265                 270

Ala Ser Ser Asp Lys Gly Ser Cys Cys Ile Val Ala Ala Gly Ser Gln
        275                 280                 285

Gly Pro Val Val Pro Ala Trp Ser Gly Pro Arg Glu Ala Pro Asp Ser
    290                 295                 300

Leu Phe Ala Val Arg Arg His Leu Trp Gly Ser His Gly Asn Ser Thr
305                 310                 315                 320

Phe Pro Glu Phe Leu His Asn Met Asp Tyr Phe Lys Phe His Asn Met
                325                 330                 335

Arg Pro Pro Phe Thr Tyr Ala Thr Leu Ile Arg Trp Ala Ile Leu Glu
            340                 345                 350

Ala Pro Glu Lys Gln Arg Thr Leu Asn Glu Ile Tyr His Trp Phe Thr
        355                 360                 365

Arg Met Phe Ala Phe Phe Arg Asn His Pro Ala Thr Trp Lys Asn Ala
    370                 375                 380

Ile Arg His Asn Leu Ser Leu His Lys Cys Phe Val Arg Val Glu Ser
385                 390                 395                 400

Glu Lys Gly Ala Val Trp Thr Val Asp Glu Leu Glu Phe Arg Lys Lys
                405                 410                 415

Arg Ser Gln Arg Pro Ser Arg Cys Ser Asn Pro Thr Pro Gly Pro Glu
            420                 425                 430

Ser Gly Gly Gly Gly Ser Pro Gly Arg Arg Arg Arg Arg Arg Arg Arg
        435                 440                 445

Arg Arg Arg
    450

<210>  23
<211>  400
<212>  PRT
<213>  Artificial Sequence

<220>
<223>  transducible material

<400>  23

Met Gly Ser Ser His His His His His His Ser Ser Gly Leu Val Pro
1               5                   10                  15

Arg Gly Ser His Met Ala Gly His Leu Ala Ser Asp Phe Ala Phe Ser
            20                  25                  30

Pro Pro Pro Gly Gly Gly Gly Asp Gly Pro Gly Gly Pro Glu Pro Gly
        35                  40                  45

Trp Val Asp Pro Arg Thr Trp Leu Ser Phe Gln Gly Pro Pro Gly Gly
    50                  55                  60

Pro Gly Ile Gly Pro Gly Val Gly Pro Gly Ser Glu Val Trp Gly Ile
65                  70                  75                  80

Pro Pro Cys Pro Pro Pro Tyr Glu Phe Cys Gly Gly Met Ala Tyr Cys
                85                  90                  95

Gly Pro Gln Val Gly Val Gly Leu Val Pro Gln Gly Gly Leu Glu Thr
            100                 105                 110

Ser Gln Pro Glu Gly Glu Ala Gly Val Gly Val Glu Ser Asn Ser Asp
        115                 120                 125

Gly Ala Ser Pro Glu Pro Cys Thr Val Thr Pro Gly Ala Val Lys Leu
    130                 135                 140

Glu Lys Glu Lys Leu Glu Gln Asn Pro Glu Glu Ser Gln Asp Ile Lys
145                 150                 155                 160

Ala Leu Gln Lys Glu Leu Glu Gln Phe Ala Lys Leu Leu Lys Gln Lys
                165                 170                 175

Arg Ile Thr Leu Gly Tyr Thr Gln Ala Asp Val Gly Leu Thr Leu Gly
            180                 185                 190

Val Leu Phe Gly Lys Val Phe Ser Gln Thr Thr Ile Cys Arg Phe Glu
        195                 200                 205

Ala Leu Gln Leu Ser Phe Lys Asn Met Cys Lys Leu Arg Pro Leu Leu
    210                 215                 220

Gln Lys Trp Val Glu Glu Ala Asp Asn Asn Glu Asn Leu Gln Glu Ile
225                 230                 235                 240

Cys Lys Ala Glu Thr Leu Val Gln Ala Arg Lys Arg Lys Arg Thr Ser
                245                 250                 255

Ile Glu Asn Arg Val Arg Gly Asn Leu Glu Asn Leu Phe Leu Gln Cys
            260                 265                 270

Pro Lys Pro Thr Leu Gln Gln Ile Ser His Ile Ala Gln Gln Leu Gly
        275                 280                 285

Leu Glu Lys Asp Val Val Arg Val Trp Phe Cys Asn Arg Arg Gln Lys
    290                 295                 300

Gly Lys Arg Ser Ser Ser Asp Tyr Ala Gln Arg Glu Asp Phe Glu Ala
305                 310                 315                 320

Ala Gly Ser Pro Phe Ser Gly Gly Pro Val Ser Phe Pro Leu Ala Pro
                325                 330                 335

Gly Pro His Phe Gly Thr Pro Gly Tyr Gly Ser Pro His Phe Thr Ala
            340                 345                 350

Leu Tyr Ser Ser Val Pro Phe Pro Glu Gly Glu Ala Phe Pro Pro Val
        355                 360                 365

Ser Val Thr Thr Leu Gly Ser Pro Met His Ser Asn Glu Ser Gly Gly
    370                 375                 380

Gly Gly Ser Pro Gly Arg Arg Arg Arg Arg Arg Arg Arg Arg Arg Arg
385                 390                 395                 400

<210>  24
<211>  357
<212>  PRT
<213>  Artificial Sequence

<220>
<223>  transducible material

<400>  24
   Met Gly Ser Ser His His His His His His Ser Ser Gly Leu Val Pro
   1               5                   10                  15
   Arg Gly Ser His Met Tyr Asn Met Met Glu Thr Glu Leu Lys Pro Pro
               20                  25                  30
   Gly Pro Gln Gln Thr Ser Gly Gly Gly Gly Gly Asn Ser Thr Ala Ala
           35                  40                  45
   Ala Ala Gly Gly Asn Gln Lys Asn Ser Pro Asp Arg Val Lys Arg Pro
       50                  55                  60
   Met Asn Ala Phe Met Val Trp Ser Arg Gly Gln Arg Arg Lys Met Ala
   65                  70                  75                  80
   Gln Glu Asn Pro Lys Met His Asn Ser Glu Ile Ser Lys Arg Leu Gly
                   85                  90                  95
   Ala Glu Trp Lys Leu Leu Ser Glu Thr Glu Lys Arg Pro Phe Ile Asp
               100                 105                 110
   Glu Ala Lys Arg Leu Arg Ala Leu His Met Lys Glu His Pro Asp Tyr
           115                 120                 125
   Lys Tyr Arg Pro Arg Arg Lys Thr Lys Thr Leu Met Lys Lys Asp Lys
       130                 135                 140
   Tyr Thr Leu Pro Gly Gly Leu Leu Ala Pro Gly Gly Asn Ser Met Ala
   145                 150                 155                 160
   Ser Gly Val Gly Val Gly Ala Gly Leu Gly Ala Gly Val Asn Gln Arg
                   165                 170                 175
   Met Asp Ser Tyr Ala His Met Asn Gly Trp Ser Asn Gly Ser Tyr Ser
               180                 185                 190
   Met Met Gln Asp Gln Leu Gly Tyr Pro Gln His Pro Gly Leu Asn Ala
           195                 200                 205
   His Gly Ala Ala Gln Met Gln Pro Met His Arg Tyr Asp Val Ser Ala
       210                 215                 220
   Leu Gln Tyr Asn Ser Met Thr Ser Ser Gln Thr Tyr Met Asn Gly Ser
   225                 230                 235                 240
   Pro Thr Tyr Ser Met Ser Tyr Ser Gln Gln Gly Thr Pro Gly Met Ala
                   245                 250                 255
   Leu Gly Ser Met Gly Ser Val Val Lys Ser Glu Ala Ser Ser Ser Pro
               260                 265                 270
   Pro Val Val Thr Ser Ser Ser His Ser Arg Ala Pro Cys Gln Ala Gly
           275                 280                 285
   Asp Leu Arg Asp Met Ile Ser Met Tyr Leu Pro Gly Ala Glu Val Pro
       290                 295                 300
   Glu Pro Ala Ala Pro Ser Arg Leu His Met Ser Gln His Tyr Gln Ser
   305                 310                 315                 320
   Gly Pro Val Pro Gly Thr Ala Ile Asn Gly Thr Leu Pro Leu Ser His
                   325                 330                 335
   Met Glu Ser Gly Gly Gly Gly Ser Pro Gly Arg Arg Arg Arg Arg Arg
               340                 345                 350
   Arg Arg Arg Arg Arg
           355

<210>  25
<211>  510
<212>  PRT
<213>  Artificial Sequence

<220>
<223>  transducible material

<400>  25
   Met Gly Ser Ser His His His His His His Ser Ser Gly Leu Val Pro
   1               5                   10                  15
   Arg Gly Ser His Met Ala Val Ser Asp Ala Leu Leu Pro Ser Phe Ser
               20                  25                  30
   Thr Phe Ala Ser Gly Pro Ala Gly Arg Glu Lys Thr Leu Arg Gln Ala
           35                  40                  45
   Gly Ala Pro Asn Asn Arg Trp Arg Glu Glu Leu Ser His Met Lys Arg
       50                  55                  60
   Leu Pro Pro Val Leu Pro Gly Arg Pro Tyr Asp Leu Ala Ala Ala Thr
   65                  70                  75                  80
   Val Ala Thr Asp Leu Glu Ser Gly Gly Ala Gly Ala Ala Cys Gly Gly
                   85                  90                  95
   Ser Asn Leu Ala Pro Leu Pro Arg Arg Glu Thr Glu Glu Phe Asn Asp
               100                 105                 110
   Leu Leu Asp Leu Asp Phe Ile Leu Ser Asn Ser Leu Thr His Pro Pro
           115                 120                 125
   Glu Ser Val Ala Ala Thr Val Ser Ser Ser Ala Ser Ala Ser Ser Ser
       130                 135                 140
   Ser Ser Pro Ser Ser Ser Gly Pro Ala Ser Ala Pro Ser Thr Cys Ser
   145                 150                 155                 160
   Phe Thr Tyr Pro Ile Arg Ala Gly Asn Asp Pro Gly Val Ala Pro Gly
                   165                 170                 175
   Gly Thr Gly Gly Gly Leu Leu Tyr Gly Arg Glu Ser Ala Pro Pro Pro
   180 185 190
   Thr Ala Pro Phe Asn Leu Ala Asp Ile Asn Asp Val Ser Pro Ser Gly
   195 200 205
   Gly Phe Val Ala Glu Leu Leu Arg Pro Glu Leu Asp Pro Val Tyr Ile
   210 215 220
   Pro Pro Gln Gln Pro Gln Pro Pro Gly Gly Gly Leu Met Gly Lys Phe
   225 230 235 240
   Val Leu Lys Ala Ser Leu Ser Ala Pro Gly Ser Glu Tyr Gly Ser Pro
   245 250 255
   Ser Val Ile Ser Val Ser Lys Gly Ser Pro Asp Gly Ser His Pro Val
   260 265 270
   Val Val Ala Pro Tyr Asn Gly Gly Pro Pro Arg Thr Cys Pro Lys Ile
   275 280 285
   Lys Gln Glu Ala Val Ser Ser Cys Thr His Leu Gly Ala Gly Pro Pro
   290 295 300
   Leu Ser Asn Gly His Arg Pro Ala Ala His Asp Phe Pro Leu Gly Arg
   305 310 315 320
   Gln Leu Pro Ser Arg Thr Thr Pro Thr Leu Gly Leu Glu Glu Val Leu
   325 330 335
   Ser Ser Arg Asp Cys His Pro Ala Leu Pro Leu Pro Pro Gly Phe His
   340 345 350
   Pro His Pro Gly Pro Asn Tyr Pro Ser Phe Leu Pro Asp Gln Met Gln
   355 360 365
   Pro Gln Val Pro Pro Leu His Tyr Gln Glu Leu Met Pro Pro Gly Ser
   370 375 380
   Cys Met Pro Glu Glu Pro Lys Pro Lys Arg Gly Arg Arg Ser Trp Pro
   385 390 395 400
   Arg Lys Arg Thr Ala Thr His Thr Cys Asp Tyr Ala Gly Cys Gly Lys
   405 410 415
   Thr Tyr Thr Lys Ser Ser His Leu Lys Ala His Leu Arg Thr His Thr
   420 425 430
   Gly Glu Lys Pro Tyr His Cys Asp Trp Asp Gly Cys Gly Trp Lys Phe
           435                 440                 445
   Ala Arg Ser Asp Glu Leu Thr Arg His Tyr Arg Lys His Thr Gly His
       450                 455                 460
   Arg Pro Phe Gln Cys Gln Lys Cys Asp Arg Ala Phe Ser Arg Ser Asp
   465                 470                 475                 480
   His Leu Ala Leu His Met Lys Arg His Phe Glu Ser Gly Gly Gly Gly
                   485                 490                 495
   Ser Pro Gly Arg Arg Arg Arg Arg Arg Arg Arg Arg Arg Arg
               500                 505                 510

<210>  26
<211>  494
<212>  PRT
<213>  Artificial Sequence

<220>
<223>  transducible material

<400>  26

Met Gly Ser Ser His His His His His His Ser Ser Gly Leu Val Pro
1               5                   10                  15

Arg Gly Ser His Met Asp Phe Phe Arg Val Val Glu Asn Gln Gln Pro
            20                  25                  30

Pro Ala Thr Met Pro Leu Asn Val Ser Phe Thr Asn Arg Asn Tyr Asp
        35                  40                  45

Leu Asp Tyr Asp Ser Val Gln Pro Tyr Phe Tyr Cys Asp Glu Glu Glu
    50                  55                  60

Asn Phe Tyr Gln Gln Gln Gln Gln Ser Glu Leu Gln Pro Pro Ala Pro
65                  70                  75                  80

Ser Glu Asp Ile Trp Lys Lys Phe Glu Leu Leu Pro Thr Pro Pro Leu
                85                  90                  95

Ser Pro Ser Arg Arg Ser Gly Leu Cys Ser Pro Ser Tyr Val Ala Val
            100                 105                 110

Thr Pro Phe Ser Leu Arg Gly Asp Asn Asp Gly Gly Gly Gly Ser Phe
        115                 120                 125

Ser Thr Ala Asp Gln Leu Glu Met Val Thr Glu Leu Leu Gly Gly Asp
    130                 135                 140

Met Val Asn Gln Ser Phe Ile Cys Asp Pro Asp Asp Glu Thr Phe Ile
145                 150                 155                 160

Lys Asn Ile Ile Ile Gln Asp Cys Met Trp Ser Gly Phe Ser Ala Ala
                165                 170                 175

Ala Lys Leu Val Ser Glu Lys Leu Ala Ser Tyr Gln Ala Ala Arg Lys
            180                 185                 190

Asp Ser Gly Ser Pro Asn Pro Ala Arg Gly His Ser Val Cys Ser Thr
        195                 200                 205

Ser Ser Leu Tyr Leu Gln Asp Leu Ser Ala Ala Ala Ser Glu Cys Ile
    210                 215                 220

Asp Pro Ser Val Val Phe Pro Tyr Pro Leu Asn Asp Ser Ser Ser Pro
225                 230                 235                 240

Lys Ser Cys Ala Ser Gln Asp Ser Ser Ala Phe Ser Pro Ser Ser Asp
                245                 250                 255

Ser Leu Leu Ser Ser Thr Glu Ser Ser Pro Gln Gly Ser Pro Glu Pro
            260                 265                 270

Leu Val Leu His Glu Glu Thr Pro Pro Thr Thr Ser Ser Asp Ser Glu
        275                 280                 285

Glu Glu Gln Glu Asp Glu Glu Glu Ile Asp Val Val Ser Val Glu Lys
    290                 295                 300

Arg Gln Ala Pro Gly Lys Arg Ser Glu Ser Gly Ser Pro Ser Ala Gly
305                 310                 315                 320

Gly His Ser Lys Pro Pro His Ser Pro Leu Val Leu Lys Arg Cys His
                325                 330                 335

Val Ser Thr His Gln His Asn Tyr Ala Ala Pro Pro Ser Thr Arg Lys
            340                 345                 350

Asp Tyr Pro Ala Ala Lys Arg Val Lys Leu Asp Ser Val Arg Val Leu
        355                 360                 365

Arg Gln Ile Ser Asn Asn Arg Lys Cys Thr Ser Pro Arg Ser Ser Asp
    370                 375                 380

Thr Glu Glu Asn Val Lys Arg Arg Thr His Asn Val Leu Glu Arg Gln
385                 390                 395                 400

Arg Arg Asn Glu Leu Lys Arg Ser Phe Phe Ala Leu Arg Asp Gln Ile
                405                 410                 415

Pro Glu Leu Glu Asn Asn Glu Lys Ala Pro Lys Val Val Ile Leu Lys
            420                 425                 430

Lys Ala Thr Ala Tyr Ile Leu Ser Val Gln Ala Glu Glu Gln Lys Leu
        435                 440                 445

Ile Ser Glu Glu Asp Leu Leu Arg Lys Arg Arg Glu Gln Leu Lys His
    450                 455                 460

Lys Leu Glu Gln Leu Arg Asn Ser Cys Ala Glu Ser Gly Gly Gly Gly
465                 470                 475                 480

Ser Pro Gly Arg Arg Arg Arg Arg Arg Arg Arg Arg Arg Arg
                485                 490

<210>  27
<211>  249
<212>  PRT
<213>  Artificial Sequence

<220>
<223>  transducible material

<400>  27

Met Gly Ser Ser His His His His His His Ser Ser Gly Leu Val Pro
1               5                   10                  15

Arg Gly Ser His Met Gly Ser Val Ser Asn Gln Gln Phe Ala Gly Gly
            20                  25                  30

Cys Ala Lys Ala Ala Glu Glu Ala Pro Glu Glu Ala Pro Glu Asp Ala
        35                  40                  45

Ala Arg Ala Ala Asp Glu Pro Gln Leu Leu His Gly Ala Gly Ile Cys
    50                  55                  60

Lys Trp Phe Asn Val Arg Met Gly Phe Gly Phe Leu Ser Met Thr Ala
65                  70                  75                  80

Arg Ala Gly Val Ala Leu Asp Pro Pro Val Asp Val Phe Val His Gln
                85                  90                  95

Ser Lys Leu His Met Glu Gly Phe Arg Ser Leu Lys Glu Gly Glu Ala
            100                 105                 110

Val Glu Phe Thr Phe Lys Lys Ser Ala Lys Gly Leu Glu Ser Ile Arg
        115                 120                 125

Val Thr Gly Pro Gly Gly Val Phe Cys Ile Gly Ser Glu Arg Arg Pro
    130                 135                 140

Lys Gly Lys Ser Met Gln Lys Arg Arg Ser Lys Gly Asp Arg Cys Tyr
145                 150                 155                 160

Asn Cys Gly Gly Leu Asp His His Ala Lys Glu Cys Lys Leu Pro Pro
                165                 170                 175

Gln Pro Lys Lys Cys His Phe Cys Gln Ser Ile Ser His Met Val Ala
            180                 185                 190

Ser Cys Pro Leu Lys Ala Gln Gln Gly Pro Ser Ala Gln Gly Lys Pro
        195                 200                 205

Thr Tyr Phe Arg Glu Glu Glu Glu Glu Ile His Ser Pro Thr Leu Leu
    210                 215                 220

Pro Glu Ala Gln Asn Glu Ser Gly Gly Gly Gly Ser Pro Gly Arg Arg
225                 230                 235                 240

Arg Arg Arg Arg Arg Arg Arg Arg Arg
                245

<210>  28
<211>  345
<212>  PRT
<213>  Artificial Sequence

<220>
<223>  transducible material

<400>  28

Met Gly Ser Ser His His His His His His Ser Ser Gly Leu Val Pro
1               5                   10                  15

Arg Gly Ser His Met Ser Val Asp Pro Ala Cys Pro Gln Ser Leu Pro
            20                  25                  30

Cys Phe Glu Ala Ser Asp Cys Lys Glu Ser Ser Pro Met Pro Val Ile
        35                  40                  45

Cys Gly Pro Glu Glu Asn Tyr Pro Ser Leu Gln Met Ser Ser Ala Glu

50                  55                  60

Met Pro His Thr Glu Thr Val Ser Pro Leu Pro Ser Ser Met Asp Leu
65                  70                  75                  80

Leu Ile Gln Asp Ser Pro Asp Ser Ser Thr Ser Pro Lys Gly Lys Gln
                85                  90                  95

Pro Thr Ser Ala Glu Asn Ser Val Ala Lys Lys Glu Asp Lys Val Pro
            100                 105                 110

Val Lys Lys Gln Lys Thr Arg Thr Val Phe Ser Ser Thr Gln Leu Cys
        115                 120                 125

Val Leu Asn Asp Arg Phe Gln Arg Gln Lys Tyr Leu Ser Leu Gln Gln
    130                 135                 140

Met Gln Glu Leu Ser Asn Ile Leu Asn Leu Ser Tyr Lys Gln Val Lys
145                 150                 155                 160

Thr Trp Phe Gln Asn Gln Arg Met Lys Ser Lys Arg Trp Gln Lys Asn
                165                 170                 175

Asn Trp Pro Lys Asn Ser Asn Gly Val Thr Gln Lys Ala Ser Ala Pro
            180                 185                 190

Thr Tyr Pro Ser Leu Tyr Ser Ser Tyr His Gln Gly Cys Leu Val Asn
        195                 200                 205

Pro Thr Gly Asn Leu Pro Met Trp Ser Asn Gln Thr Trp Asn Asn Ser
    210                 215                 220

Thr Trp Ser Asn Gln Thr Gln Asn Ile Gln Ser Trp Ser Asn His Ser
225                 230                 235                 240

Trp Asn Thr Gln Thr Trp Cys Thr Gln Ser Trp Asn Asn Gln Ala Trp
                245                 250                 255

Asn Ser Pro Phe Tyr Asn Cys Gly Glu Glu Ser Leu Gln Ser Cys Met
            260                 265                 270

Gln Phe Gln Pro Asn Ser Pro Ala Ser Asp Leu Glu Ala Ala Leu Glu
        275                 280                 285

Ala Ala Gly Glu Gly Leu Asn Val Ile Gln Gln Thr Thr Arg Tyr Phe
    290                 295                 300

Ser Thr Pro Gln Thr Met Asp Leu Phe Leu Asn Tyr Ser Met Asn Met
305                 310                 315                 320

Gln Pro Glu Asp Val Glu Ser Gly Gly Gly Gly Ser Pro Gly Arg Arg
                325                 330                 335

Arg Arg Arg Arg Arg Arg Arg Arg Arg
            340                 345

<210>  29
<211>  396
<212>  PRT
<213>  Artificial Sequence

<220>
<223>  transducible material

<400>  29

Met Gly Ser Ser His His His His His His Ser Ser Gly Leu Val Pro
1               5                   10                  15

Arg Gly Ser His Met Thr Lys Ser Tyr Ser Glu Ser Gly Leu Met Gly
            20                  25                  30

Glu Pro Gln Pro Gln Gly Pro Pro Ser Trp Thr Asp Glu Cys Leu Ser
        35                  40                  45

Ser Gln Asp Glu Glu His Glu Ala Asp Lys Lys Glu Asp Asp Leu Glu
    50                  55                  60

Ala Met Asn Ala Glu Glu Asp Ser Leu Arg Asn Gly Gly Glu Glu Glu
65                  70                  75                  80

Asp Glu Asp Glu Asp Leu Glu Glu Glu Glu Glu Glu Glu Glu Glu Asp
                85                  90                  95

Asp Asp Gln Lys Pro Lys Arg Arg Gly Pro Lys Lys Lys Lys Met Thr
            100                 105                 110

Lys Ala Arg Leu Glu Arg Phe Lys Leu Arg Arg Met Lys Ala Asn Ala
        115                 120                 125

Arg Glu Arg Asn Arg Met His Gly Leu Asn Ala Ala Leu Asp Asn Leu
    130                 135                 140

Arg Lys Val Val Pro Cys Tyr Ser Lys Thr Gln Lys Leu Ser Lys Ile
145                 150                 155                 160

Glu Thr Leu Arg Leu Ala Lys Asn Tyr Ile Trp Ala Leu Ser Glu Ile

165                 170                 175

Leu Arg Ser Gly Lys Ser Pro Asp Leu Val Ser Phe Val Gln Thr Leu
            180                 185                 190

Cys Lys Gly Leu Ser Gln Pro Thr Thr Asn Leu Val Ala Gly Cys Leu
        195                 200                 205

Gln Leu Asn Pro Arg Thr Phe Leu Pro Glu Gln Asn Gln Asp Met Pro
    210                 215                 220

Pro His Leu Pro Thr Ala Ser Ala Ser Phe Pro Val His Pro Tyr Ser
225                 230                 235                 240

Tyr Gln Ser Pro Gly Leu Pro Ser Pro Pro Tyr Gly Thr Met Asp Ser
                245                 250                 255

Ser His Val Phe His Val Lys Pro Pro Pro His Ala Tyr Ser Ala Ala
            260                 265                 270

Leu Glu Pro Phe Phe Glu Ser Pro Leu Thr Asp Cys Thr Ser Pro Ser
        275                 280                 285

Phe Asp Gly Pro Leu Ser Pro Pro Leu Ser Ile Asn Gly Asn Phe Ser
    290                 295                 300

Phe Lys His Glu Pro Ser Ala Glu Phe Glu Lys Asn Tyr Ala Phe Thr
305                 310                 315                 320

Met His Tyr Pro Ala Ala Thr Leu Ala Gly Ala Gln Ser His Gly Ser
                325                 330                 335

Ile Phe Ser Gly Thr Ala Ala Pro Arg Cys Glu Ile Pro Ile Asp Asn
            340                 345                 350

Ile Met Ser Phe Asp Ser His Ser His His Glu Arg Val Met Ser Ala
        355                 360                 365

Gln Leu Asn Ala Ile Phe His Asp Glu Ser Gly Gly Gly Gly Ser Pro
    370                 375                 380

Gly Arg Arg Arg Arg Arg Arg Arg Arg Arg Arg Arg
385                 390                 395

<210>  30
<211>  254
<212>  PRT
<213>  Artificial Sequence

<220>
<223>  transducible material

<400>  30

<210>  31
<211>  323
<212>  PRT
<213>  Artificial Sequence

<220>
<223>  transducible material

<400>  31

Met Gly Ser Ser His His His His His His Ser Ser Gly Leu Val Pro
1               5                   10                  15

Arg Gly Ser His Met Asn Gly Glu Glu Gln Tyr Tyr Ala Ala Thr Gln
            20                  25                  30

Leu Tyr Lys Asp Pro Cys Ala Phe Gln Arg Gly Pro Ala Pro Glu Phe
        35                  40                  45

Ser Ala Ser Pro Pro Ala Cys Leu Tyr Met Gly Arg Gln Pro Pro Pro
    50                  55                  60

Pro Pro Pro His Pro Phe Pro Gly Ala Leu Gly Ala Leu Glu Gln Gly
65                  70                  75                  80

Ser Pro Pro Asp Ile Ser Pro Tyr Glu Val Pro Pro Leu Ala Asp Asp
                85                  90                  95

Pro Ala Val Ala His Leu His His His Leu Pro Ala Gln Leu Ala Leu
            100                 105                 110

Pro His Pro Pro Ala Gly Pro Phe Pro Glu Gly Ala Glu Pro Gly Val
        115                 120                 125

Leu Glu Glu Pro Asn Arg Val Gln Leu Pro Phe Pro Trp Met Lys Ser
    130                 135                 140

Thr Lys Ala His Ala Trp Lys Gly Gln Trp Ala Gly Gly Ala Tyr Ala
145                 150                 155                 160

Ala Glu Pro Glu Glu Asn Lys Arg Thr Arg Thr Ala Tyr Thr Arg Ala
                165                 170                 175

Gln Leu Leu Glu Leu Glu Lys Glu Phe Leu Phe Asn Lys Tyr Ile Ser
            180                 185                 190

Arg Pro Arg Arg Val Glu Leu Ala Val Met Leu Asn Leu Thr Glu Arg
        195                 200                 205

His Ile Lys Ile Trp Phe Gln Asn Arg Arg Met Lys Trp Lys Lys Glu
    210                 215                 220

Glu Asp Lys Lys Arg Gly Gly Gly Thr Ala Val Gly Gly Gly Gly Val
225                 230                 235                 240

Ala Glu Pro Glu Gln Asp Cys Ala Val Thr Ser Gly Glu Glu Leu Leu
                245                 250                 255

Ala Leu Pro Pro Pro Pro Pro Pro Gly Gly Ala Val Pro Pro Ala Ala
            260                 265                 270

Pro Val Ala Ala Arg Glu Gly Arg Leu Pro Pro Gly Leu Ser Ala Ser
        275                 280                 285

Pro Gln Pro Ser Ser Val Ala Pro Arg Arg Pro Gln Glu Pro Arg Glu
    290                 295                 300

Ser Gly Gly Gly Gly Ser Pro Gly Arg Arg Arg Arg Arg Arg Arg Arg
305                 310                 315                 320

Arg Arg Arg

<210>  32
<211>  392
<212>  PRT
<213>  Artificial Sequence

<220>
<223>  transducible material

<400>  32

Met Gly Ser Ser His His His His His His Ser Ser Gly Leu Val Pro
1               5                   10                  15

Arg Gly Ser His Met Ala Ala Glu Leu Ala Met Gly Ala Glu Leu Pro
            20                  25                  30

Ser Ser Pro Leu Ala Ile Glu Tyr Val Asn Asp Phe Asp Leu Met Lys
        35                  40                  45

Phe Glu Val Lys Lys Glu Pro Pro Glu Ala Glu Arg Phe Cys His Arg
    50                  55                  60

Leu Pro Pro Gly Ser Leu Ser Ser Thr Pro Leu Ser Thr Pro Cys Ser
65                  70                  75                  80

Ser Val Pro Ser Ser Pro Ser Phe Cys Ala Pro Ser Pro Gly Thr Gly
                85                  90                  95

Gly Gly Gly Gly Ala Gly Gly Gly Gly Gly Ser Ser Gln Ala Gly Gly
            100                 105                 110

Ala Pro Gly Pro Pro Ser Gly Gly Pro Gly Ala Val Gly Gly Thr Ser
        115                 120                 125

Gly Lys Pro Ala Leu Glu Asp Leu Tyr Trp Met Ser Gly Tyr Gln His
    130                 135                 140

His Leu Asn Pro Glu Ala Leu Asn Leu Thr Pro Glu Asp Ala Val Glu
145                 150                 155                 160

Ala Leu Ile Gly Ser Gly His His Gly Ala His His Gly Ala His His
                165                 170                 175

Pro Ala Ala Ala Ala Ala Tyr Glu Ala Phe Arg Gly Pro Gly Phe Ala
            180                 185                 190

Gly Gly Gly Gly Ala Asp Asp Met Gly Ala Gly His His His Gly Ala
        195                 200                 205

His His Ala Ala His His His His Ala Ala His His His His His His
    210                 215                 220

His His His Gly Gly Ala Gly His Gly Gly Gly Ala Gly His His Val
225                 230                 235                 240

Arg Leu Glu Glu Arg Phe Ser Asp Asp Gln Leu Val Ser Met Ser Val
                245                 250                 255

Arg Glu Leu Asn Arg Gln Leu Arg Gly Phe Ser Lys Glu Glu Val Ile
            260                 265                 270

Arg Leu Lys Gln Lys Arg Arg Thr Leu Lys Asn Arg Gly Tyr Ala Gln
        275                 280                 285

Ser Cys Arg Phe Lys Arg Val Gln Gln Arg His Ile Leu Glu Ser Glu
    290                 295                 300

Lys Cys Gln Leu Gln Ser Gln Val Glu Gln Leu Lys Leu Glu Val Gly
305                 310                 315                 320

Arg Leu Ala Lys Glu Arg Asp Leu Tyr Lys Glu Lys Tyr Glu Lys Leu
                325                 330                 335

Ala Gly Arg Gly Gly Pro Gly Ser Ala Gly Gly Ala Gly Phe Pro Arg
            340                 345                 350

Glu Pro Ser Pro Pro Gln Ala Gly Pro Gly Gly Ala Lys Gly Thr Ala
        355                 360                 365

Asp Phe Phe Leu Glu Ser Gly Gly Gly Gly Ser Pro Gly Arg Arg Arg
    370                 375                 380

Arg Arg Arg Arg Arg Arg Arg Arg
385                 390

<210> 33
<211> 471
<212> PRT
<213> Artificial Sequence

<220>
<223> transducible material

<400> 33

Met Gly Ser Ser His His His His His His Ser Ser Gly Leu Val Pro
1               5                   10                  15

Arg Gly Ser His Met Pro Asn Pro Arg Pro Gly Lys Pro Ser Ala Pro
            20                  25                  30

Ser Leu Ala Leu Gly Pro Ser Pro Gly Ala Ser Pro Ser Trp Arg Ala
        35                  40                  45

Ala Pro Lys Ala Ser Asp Leu Leu Gly Ala Arg Gly Pro Gly Gly Thr
    50                  55                  60

Phe Gln Gly Arg Asp Leu Arg Gly Gly Ala His Ala Ser Ser Ser Ser
65                  70                  75                  80

Leu Asn Pro Met Pro Pro Ser Gln Leu Gln Leu Pro Thr Leu Pro Leu
                85                  90                  95

Val Met Val Ala Pro Ser Gly Ala Arg Leu Gly Pro Leu Pro His Leu
            100                 105                 110

Gln Ala Leu Leu Gln Asp Arg Pro His Phe Met His Gln Leu Ser Thr
        115                 120                 125

Val Asp Ala His Ala Arg Thr Pro Val Leu Gln Val His Pro Leu Glu
    130                 135                 140

Ser Pro Ala Met Ile Ser Leu Thr Pro Pro Thr Thr Ala Thr Gly Val
145                 150                 155                 160

Phe Ser Leu Lys Ala Arg Pro Gly Leu Pro Pro Gly Ile Asn Val Ala
                165                 170                 175

Ser Leu Glu Trp Val Ser Arg Glu Pro Ala Leu Leu Cys Thr Phe Pro
            180                 185                 190

Asn Pro Ser Ala Pro Arg Lys Asp Ser Thr Leu Ser Ala Val Pro Gln
        195                 200                 205

Ser Ser Tyr Pro Leu Leu Ala Asn Gly Val Cys Lys Trp Pro Gly Cys
    210                 215                 220

Glu Lys Val Phe Glu Glu Pro Glu Asp Phe Leu Lys His Cys Gln Ala
225                 230                 235                 240

Asp His Leu Leu Asp Glu Lys Gly Arg Ala Gln Cys Leu Leu Gln Arg
                245                 250                 255

Glu Met Val Gln Ser Leu Glu Gln Gln Leu Val Leu Glu Lys Glu Lys
            260                 265                 270

Leu Ser Ala Met Gln Ala His Leu Ala Gly Lys Met Ala Leu Thr Lys
        275                 280                 285

Ala Ser Ser Val Ala Ser Ser Asp Lys Gly Ser Cys Cys Ile Val Ala
    290                 295                 300

Ala Gly Ser Gln Gly Pro Val Val Pro Ala Trp Ser Gly Pro Arg Glu
305                 310                 315                 320

Ala Pro Asp Ser Leu Phe Ala Val Arg Arg His Leu Trp Gly Ser His
                325                 330                 335

Gly Asn Ser Thr Phe Pro Glu Phe Leu His Asn Met Asp Tyr Phe Lys
            340                 345                 350

Phe His Asn Met Arg Pro Pro Phe Thr Tyr Ala Thr Leu Ile Arg Trp
        355                 360                 365

Ala Ile Leu Glu Ala Pro Glu Lys Gln Arg Thr Leu Asn Glu Ile Tyr
    370                 375                 380

His Trp Phe Thr Arg Met Phe Ala Phe Phe Arg Asn His Pro Ala Thr
385                 390                 395                 400

Trp Lys Asn Ala Ile Arg His Asn Leu Ser Leu His Lys Cys Phe Val
                405                 410                 415

Arg Val Glu Ser Glu Lys Gly Ala Val Trp Thr Val Asp Glu Leu Glu
            420                 425                 430

Phe Arg Lys Lys Arg Ser Gln Arg Pro Ser Arg Cys Ser Asn Pro Thr
        435                 440                 445

Pro Gly Pro Glu Ser Gly Gly Gly Gly Ser Pro Gly Arg Arg Arg Arg
    450                 455                 460

Arg Arg Arg Arg Arg Arg Arg
465                 470

<210>  34
<211>  11
<212>  PRT
<213>  Human immunodeficiency virus type 1

<400>  34

Tyr Gly Arg Lys Lys Arg Arg Gln Arg Arg Arg
1               5                   10

<210>  35
<211>  10
<212>  PRT
<213>  Artificial Sequence

<220>
<223>  protein transducible domain

<400>  35

Arg Arg Arg Arg Arg Arg Arg Arg Arg Arg
1               5                   10

<210>  36
<211>  11
<212>  PRT
<213>  Artificial Sequence

<220>
<223>  protein transducible domain

<400>  36

Arg Arg Arg Arg Arg Arg Arg Arg Arg Arg Arg
1               5                   10

<210>  37
<211>  20
<212>  PRT
<213>  Artificial Sequence

<220>
<223>  protein transducible domain

<400>  37

Glu Ser Gly Gly Gly Gly Ser Pro Gly Arg Arg Arg Arg Arg Arg Arg
1               5                   10                  15

Arg Arg Arg Arg
            20

<210>  38
<211>  16
<212>  PRT
<213>  Drosophila melanogaster

<400>  38

Arg Gln Ile Lys Ile Trp Phe Gln Asn Arg Arg Met Lys Trp Lys Lys
1               5                   10                  15

<210>  39
<211>  34
<212>  PRT
<213>  herpes simplex virus 7

<400>  39

Asp Ala Ala Thr Ala Thr Arg Gly Arg Ser Ala Ala Ser Arg Pro Thr
1               5                   10                  15

Gln Arg Pro Arg Ala Pro Ala Arg Ser Ala Ser Arg Pro Arg Arg Pro
            20                  25                  30

Val Gln

<210>  40
<211>  27
<212>  PRT
<213>  Artificial Sequence

<220>
<223>  protein transducible domain

<400>  40

Gly Trp Thr Leu Asn Ser Ala Gly Tyr Leu Leu Gly Lys Ile Asn Leu
1               5                   10                  15

Lys Ala Leu Ala Ala Leu Ala Lys Lys Ile Leu
            20                  25

<210>  41
<211>  18
<212>  PRT
<213>  Artificial Sequence

<220>
<223>  protein transducible domain

<400>  41

Lys Leu Ala Leu Lys Leu Ala Leu Lys Ala Leu Lys Ala Ala Leu Lys
1               5                   10                  15

Leu Ala

<210>  42
<211>  15
<212>  PRT
<213>  Artificial Sequence

<220>
<223>  protein transducible domain

<400>  42

Ala Ala Val Ala Leu Leu Pro Ala Val Leu Leu Ala Leu Leu Pro
1               5                   10                  15

<210>  43
<211>  21
<212>  PRT
<213>  Artificial Sequence

<220>
<223>  protein transducible domain

<400>  43

Lys Glu Thr Trp Trp Glu Thr Trp Trp Thr Glu Trp Ser Gln Pro Lys
1               5                   10                  15

Lys Lys Arg Lys Val
            20

<210>  44
<211>  14
<212>  PRT
<213>  Artificial Sequence

<220>
<223>  protein transducible domain

<400>  44

Arg Arg Trp Arg Arg Trp Trp Arg Arg Trp Trp Arg Arg Trp
1               5                   10


<210>  45
<211>  12
<212>  PRT
<213>  Artificial Sequence

<220>
<223>  cell-targeting peptide

<400>  45

Thr Ser Pro Leu Asn Ile His Asn Gly Gln Lys Leu
1               5                   10


<210>  46
<211>  5
<212>  PRT
<213>  Artificial Sequence

<220>
<223>  cell-targeting peptide

<400>  46

Cys Gly Lys Arg Lys
1               5


<210>  47
<211>  9
<212>  PRT
<213>  Artificial Sequence

<220>
<223>  cell-targeting peptide

<400>  47

Cys Gly Asn Lys Arg Thr Arg Gly Cys
1               5


<210>  48
<211>  7
<212>  PRT
<213>  Artificial Sequence

<220>
<223>  cell-targeting peptide

<400>  48

Ser Met Ser Ile Ala Arg Leu
1               5


<210>  49

<211>  7
<212>  PRT
<213>  Artificial Sequence

<220>
<223>  cell-targeting peptide

<400>  49

Phe Gln His Pro Ser Phe Ile
1               5


<210>  50
<211>  7
<212>  PRT
<213>  Artificial Sequence

<220>
<223>  cell-targeting peptide

<400>  50

Val His Ser Pro Asn Lys Lys
1               5


<210>  51
<211>  6
<212>  PRT
<213>  Artificial Sequence

<220>
<223>  cell-targeting peptide

<400>  51

Arg Arg Pro Tyr Ile Leu
1               5


<210>  52
<211>  12
<212>  PRT
<213>  Artificial Sequence

<220>
<223>  cell-targeting peptide

<400>  52

Glu Asp Tyr Glu Leu Met Asp Leu Leu Ala Tyr Leu
1               5                   10


<210>  53
<211>  7
<212>  PRT
<213>  Artificial Sequence

<220>
<223>  cell-targeting peptide

<400>  53

Leu Thr Val Ser Pro Trp Tyr
1               5

<210>  54
<211>  7
<212>  PRT
<213>  Artificial Sequence

<220>
<223>  cell-targeting peptide

<400>  54

Ala Thr Trp Leu Pro Pro Arg
1               5

<210>  55
<211>  9
<212>  PRT
<213>  Artificial Sequence

<220>
<223>  linker

<400>  55

Glu Ser Gly Gly Gly Gly Ser Pro Gly
1               5

<210>  56
<211>  16
<212>  PRT
<213>  Homo sapiens

<400>  56

Arg His Ile Lys Ile Trp Phe Gln Asn Arg Arg Met Lys Trp Lys Lys
1               5                   10                  15

<210>  57
<211>  34
<212>  PRT
<213>  Homo sapiens

<400>  57

Lys Pro Lys Arg Arg Gly Pro Lys Lys Lys Lys Met Thr Lys Ala Arg
1               5                   10                  15

Leu Glu Arg Phe Lys Leu Arg Arg Met Lys Ala Asn Ala Arg Glu Arg
            20                  25                  30

Asn Arg

<210>  58
<211>  10
<212>  PRT
<213>  Homo sapiens

<400>  58

Glu His Trp Ser Tyr Gly Leu Arg Pro Gly
1               5                   10


<210>  59
<211>  20
<212>  PRT
<213>  Artificial Sequence

<220>
<223>  protein purification

<400>  59

Met Gly Ser Ser His His His His His His Ser Ser Gly Leu Val Pro
1               5                   10                  15


Arg Gly Ser His
            20


<210>  60
<211>  17
<212>  PRT
<213>  Human immunodeficiency virus type 1

<400>  60

Thr Arg Gln Ala Arg Arg Asn Arg Arg Arg Arg Trp Arg Glu Arg Gln
1               5                   10                  15


Arg




<210>  61
<211>  15
<212>  PRT
<213>  flock house virus

<400>  61

Arg Arg Arg Arg Asn Arg Thr Arg Arg Asn Arg Arg Arg Val Arg
1               5                   10                  15


<210>  62
<211>  26
<212>  PRT
<213>  Homo sapiens

<400>  62

Lys Arg Arg Ile Arg Arg Glu Arg Asn Lys Met Ala Ala Ala Lys Ser
1               5                   10                  15

Arg Asn Arg Arg Arg Glu Leu Thr Asp Thr
            20                  25

<210>  63
<211>  28
<212>  PRT
<213>  Homo sapiens

<400>  63

Arg Ile Lys Ala Glu Arg Lys Arg Met Arg Asn Arg Ile Ala Ala Ser
1               5                   10                  15

Lys Ser Arg Lys Arg Lys Leu Glu Arg Ile Ala Arg
            20                  25

<210>  64
<211>  22
<212>  PRT
<213>  Saccharomyces cerevisiae

<400>  64

Lys Arg Ala Arg Asn Thr Glu Ala Ala Arg Arg Ser Arg Ala Arg Lys
1               5                   10                  15

Leu Gln Arg Met Lys Gln
            20